# EUROPEAN PATENT SPECIFICATION

(11) **EP 1 317 476 B1**
(45) Date of publication and mention of the grant of the patent: **02.01.2008**
(21) Application number: 01941591.8
(22) Date of filing: 23.05.2001
(51) Int. Cl.: C07K 7/06, C07K 7/08, C07K 1/04, G01N 33/569, G01N 33/574, G01N 33/543, A61P 37/06, C12N 5/06

(54) **REAGENTS FOR CELL SELECTION AND METHODS OF USE**
REAGENZIEN ZUR ZELLSELEKTION UND VERFAHREN ZUR ANWENDUNG
REACTIFS POUR SELECTION DE CELLULES ET MODES D'UTILISATION

(30) Priority: 23.05.2000 US 578784; 11.09.2000 US 659469
(43) Date of publication of application: 11.06.2003
(73) Proprietor: Baxter Healthcare S.A., 8304 Wallisellen (CH); Baxter International Inc., Deerfield, IL 60015 (US)
(72) Inventor: SCHAEFFER, Andrew, T., La Mirada, CA 90638 (US); TSENG-LAW, Janet, Aliso Viejo, CA 92656 (US); THORNTON, Jeffrey, R., Rancho Santa Margarita, CA 92688 (US); VAN EPPS, Dennis, E., Coto de Caza, CA 92679 (US)
(74) Representative: UEXKÜLL & STOLBERG
(86) International application number: PCT/US2001/016840
(87) International publication number: WO 2001/090153

(56) References cited:
- WO-A-91/01368
- WO-A-94/02016
- WO-A-95/07466
- WO-A-95/34817
- WO-A-99/61588
- TSENG-LAW JANET ET AL: "Identification of a peptide directed against the anti-CD34 antibody, 9C5, by phage display and its use in hematopoietic stem cell selection." EXPERIMENTAL HEMATOLOGY (CHARLOTTESVILLE), vol. 27, no. 5, May 1999 (1999-05), pages 936-945, XP002192619 ISSN: 0301-472X cited in the application
- SCHUCHERT M J ET AL: "CHARACTERIZATION OF A NEWLY DISCOVERED T-CELL RECEPTOR BETA-CHAIN HETERODIMER EXPRESSED ON A CD8+ BONE MARROW SUBPOPULATION THAT PROMOTES ALLOGENEIC STEM CELL ENGRAFTMENT" NATURE MEDICINE, NATURE PUBLISHING, CO, US, vol. 6, no. 8, August 2000 (2000-08), pages 904-909, XP002936229 ISSN: 1078-8956 cited in the application

### SEQUENCE LISTING

<110> Nexell Therapeutics Inc.
<120> Reagents For Cell Selection and Methods of Use
<130> FP-NT4664
<150> US 09/578,784
   <151> 2000-05-23
<150> US 09/659,469
   <151> 2000-09-11
<160> 57
<170> FastSEQ for Windows Version 4.0
<210> 1
   <211> 6
   <212> PRT
   <213> Artificial Sequence
<220>
   <223> synthetic peptide
<400> 1
   Gln Gln Gly Trp Phe Pro
    1               5
<210> 2
   <211> 8
   <212> PRT
   <213> Artificial Sequence
<220>
   <223> synthetic peptide
<400> 2
   Gln Gln Gly Trp Phe Pro Lys Asp
    1               5
<210> 3
   <211> 9
   <212> PRT
   <213> Artificial Sequence
<220>
   <223> synthetic peptide
<400> 3
   Gln Gln Gly Trp Phe Pro Lys Asp Cys
    1               5
<210> 4
   <211> 6
   <212> PRT
   <213> Artificial Sequence
<220>
   <223> synthetic peptide
<400> 4
   Gln Gln Gly Phe Phe Pro
    1               5
<210> 5
   <211> 6
   <212> PRT
   <213> Artificial Sequence
<220>
   <223> synthetic peptide
<400> 5
   Thr Gln Gly Thr Phe Ser
   1 5
<210> 6
   <211> 6
   <212> PRT
   <213> Artificial Sequence
<220>
   <223> synthetic peptide
<400> 6
   Asn Gln Gly Tyr Phe Pro
   1 5
<210> 7
   <211> 18
   <212> PRT
   <213> Artificial Sequence
<220>
   <223> synthetic peptide
<221> VARIANT
   <222> (1)...(18)
   <223> Xaa =any hydrophilic, polar or charged amino acid that may or may not be present
<400> 7
   Xaa Xaa Xaa Xaa Xaa Xaa Gln Gln Gly Trp Phe Pro Xaa Xaa Xaa Xaa
   1 5 10 15
   Xaa Xaa
<210> 8
   <211> 18
   <212> PRT
   <213> Artificial Sequence
<220>
   <223> synthetic peptide
<221> VARIANT
   <222> (1)...(18)
   <223> Xaa =any hydrophilc, polar or charged amino acid that may or may not be present
<400> 8
   Xaa Xaa Xaa Xaa Xaa Xaa Thr Gln Gly Ser Phe Trp Xaa Xaa Xaa Xaa
    1               5                  10                  15
   Xaa Xaa
<210> 9
   <211> 20
   <212> PRT
   <213> Artificial Sequence
<220>
   <223> synthetic peptide
<221> VARIANT
   <222> (1)...(20)
   <223> Xaa=any hyrophilic, polar, or charged amino acid that may or may not be present
<400> 9
   Xaa Xaa Xaa Xaa Xaa Xaa Gln Gln Gly Trp Phe Pro Lys Asp Xaa Xaa
    1               5                  10                  15
   Xaa Xaa Xaa Xaa
               20
<210> 10
   <211> 20
   <212> PRT
   <213> Artificial Sequence
<220>
   <223> synthetic peptide
<221> VARIANT
   <222> (1)...(20)
   <223> Xaa=any hydrophilic, polar, or charged amino acid that may of may not be present
<400> 10
   Xaa Xaa Xaa Xaa Xaa Xaa Gln Gln Gly Trp Phe Pro Asp Lys Xaa Xaa
    1               5                  10                  15
   Xaa Xaa Xaa Xaa
               20
<210> 11
   <211> 26
   <212> PRT
   <213> Artificial Sequence
<220>
   <223> synthetic peptide
<221> VARIANT
   <222> (1)...(6)
   <223> Xaa=any hydrophilic, polar or charged amino acid that may or may not be present
<221> VARIANT
   <222> 11,14,16
   <223> Xaa=any amino acid
<221> VARIANT
   <222> (21)...(26)
   <223> Xaa=any hydrophilic, polar or charged amino acid that may or may not be present
<400> 11
   Xaa Xaa Xaa Xaa Xaa Xaa Ala Asp Gly Ala Xaa Gln Gly Xaa Phe Xaa
    1               5                  10                  15
   Gly Ala Lys Asp Xaa Xaa Xaa Xaa Xaa Xaa
               20                  25
<210> 12
   <211> 26
   <212> PRT
   <213> Artificial Sequence
<220>
   <223> synthetic peptide
<221> VARIANT
   <222> (1)...(26)
   <223> Xaa=any hydrophilic, polar or charged amino acid that may or may not be present
<400> 12
   Xaa Xaa Xaa Xaa Xaa Xaa Ala Asp Gly Ala Gln Gln Gly Trp Phe Pro
    1               5                  10                  15
   Gly Ala Lys Asp Xaa Xaa Xaa Xaa Xaa Xaa
               20                  25
<210> 13
   <211> 26
   <212> PRT
   <213> Artificial Sequence
<220>
   <223> synthetic peptide
<221> VARIANT
   <222> (1)...(26)
   <223> Xaa=any hydrophilic, polar or charged amino acid that may or may not be present
<400> 13
   Xaa Xaa Xaa Xaa Xaa Xaa Ala Asp Gly Ala Thr Gln Gly Ser Phe Trp
    1               5                  10                  15
   Gly Ala Lys Asp Xaa Xaa Xaa Xaa Xaa Xaa
               20                  25
<210> 14
   <211> 18
   <212> PRT
   <213> Artificial Sequence
<220>
   <223> synthetic peptide
<221> VARIANT
   <222> (1)...(18)
   <223> Xaa=any hydrophilic, polar or charged amino acid that may or may not be present
<400> 14
   Xaa Xaa Xaa Xaa Xaa Xaa Asn Ser Ser Val Gln Ser Xaa Xaa Xaa Xaa
    1               5                  10                  15
   Xaa Xaa
<210> 15
   <211> 26
   <212> PRT
   <213> Artificial Sequence
<220>
   <223> synthetic peptide
<221> VARIANT
   <222> (1)...(26)
   <223> Xaa=any hydrophilic, polar or charged amino acid that may or may not be present
<400> 15
   Xaa Xaa Xaa Xaa Xaa Xaa Ala Asp Gly Ala Leu Ile Ser Gln Val Ser
    1               5                  10                  15
   Gly Ala Lys Asp Xaa Xaa Xaa Xaa Xaa Xaa
               20                  25
<210> 16
   <211> 18
   <212> PRT
   <213> Artificial Sequence
<220>
   <223> synthetic peptide
<221> VARIANT
   <222> (1)...(18)
   <223> Xaa=any hydrophilic, polar or charged amino acid that may or may not be present
<400> 16
   Xaa Xaa Xaa Xaa Xaa Xaa Leu Ile Ser Gln Val Ser Xaa Xaa Xaa Xaa
   1 5 10 15
   Xaa Xaa
<210> 17
   <211> 18
   <212> PRT
   <213> Artificial Sequence
<220>
   <223> synthetic peptide
<221> VARIANT
   <222> (1)...(6)
   <223> Xaa=any hydrophilic, polar or charged amino acid that may or may not be present
<221> VARIANT
   <222> 11,12
   <223> Xaa=any amino acid
<221> VARIANT
   <222> (13)...(18)
   <223> Xaa=any hydrophilic, polar or charged amino acid that may or may not be present
<400> 17
   Xaa Xaa Xaa Xaa Xaa Xaa Asn Ser Ser Val Xaa Xaa Xaa Xaa Xaa Xaa
   1 5 10 15
   Xaa Xaa
<210> 18
   <211> 18
   <212> PRT
   <213> Artificial Sequence
<220>
   <223> synthetic peptide
<221> VARIANT
   <222> (1)...(18)
   <223> Xaa=any hydrophilic, polar or charged amino acid that may or may not be present
<400> 18
   Xaa Xaa Xaa Xaa Xaa Xaa Asn Ser Ser Val Gly Leu Xaa Xaa Xaa Xaa
   1 5 10 15
   Xaa Xaa
<210> 19
   <211> 18
   <212> PRT
   <:213> Artificial Sequence
<220>
   <223> synthetic peptide
<221> VARIANT
   <222> (1)...(18)
   <223> Xaa=any hydrophilic, polar or charged amino acid that may or may not be present
<400> 19
   Xaa Xaa Xaa Xaa Xaa Xaa Thr Gly Gln Ala Ser Thr Xaa Xaa Xaa Xaa
    1               5                   10                  15
   Xaa Xaa
<210> 20
   <211> 26
   <212> PRT
   <213> Artificial Sequence
<220>
   <223> synthetic peptide
   <221> VARIANT
   <222> (1) ... (26)
   <223> Xaa=any hydrophilic, polar or charged amino acid that may or may not be present
<400> 20
   Xaa Xaa Xaa Xaa Xaa Xaa Ala Asp Gly Ala Pro Phe Trp Gly Gln Gln
    1               5                   10                  15
   Gly Ala Lys Asp Xaa Xaa Xaa Xaa Xaa Xaa
               20                  25
<210> 21
   <211> 26
   <212> PRT
   <213> Artificial Sequence
<220>
   <223> synthetic peptide
<221> VARIANT
   <222> (1)...(26)
   <223> Xaa=any hydrophilic, polar or charged amino acid that may or may not be present
<400> 21
   Xaa Xaa Xaa Xaa Xaa Xaa Ala Asp Gly Ala Thr Gln Gly Thr Phe Ser
    1               5                   10                  15
   Gly Ala Lys Asp Xaa Xaa Xaa Xaa Xaa Xaa
               20                  25
<210> 22
   <211> 27
   <212> PRT
   <213> Artificial Sequence
<220>
   <223> synthetic peptide
<221> VARIANT
   <222> (1)...(27)
   <223> Xaa=any hydrophilic, polar or charged amino acid that may or may not be present
<400> 22
   Xaa Xaa Xaa Xaa Xaa Xaa Pro Glu Leu Pro Thr Gln Gly Thr Phe Ser
    1               5                  10                  15
   Asn Val Ser Lys Glu Xaa Xaa Xaa Xaa Xaa Xaa
               20                  25
<210> 23
   <211> 26
   <212> PRT
   <213> Artificial Sequence
<220>
   <223> synthetic peptide
<221> VARIANT
   <222> (1) ... (26)
   <223> Xaa=any hydrophilic, polar or charged amino acid that may or may not be present
<400> 23
   Xaa Xaa Xaa Xaa Xaa Xaa Ala Asp Gly Ala Thr Gln Gly Ile Cys Leu
    1               5                  10                  15
   Gly Ala Lys Asp Xaa Xaa Xaa Xaa Xaa Xaa
               20                  25
<210> 24
   <211>27
   <212> PRT
   <213> Artificial Sequence
<220>
   <223> synthetic peptide
<221> VARIANT
   <222> (1)...(27)
   <223> Xaa=any hydrophilic, polar or charged amino acid that may or may not be present
<400> 24
   Xaa Xaa Xaa Xaa Xaa Xaa Glu Val Lys Leu Thr Gln Gly Ile Cys Leu
    1               5                   10                  15
   Glu Gln Asn Lys Thr Xaa Xaa Xaa Xaa Xaa Xaa
               20                  25
<210> 25
   <211> 26
   <212> PRT
   <213> Artificial Sequence
<220>
   <223> synthetic peptide
<221> VARIANT
   <222> (1)...(26)
   <223> Xaa=any hydrophilic, polar or charged amino acid that may or may not be present
<400> 25
   Xaa Xaa Xaa Xaa Xaa Xaa Ala Asp Gly Ala Asn Gln Gly Tyr Phe Pro
    1               5                   10                  15
   Gly Ala Lys Asp Xaa Xaa Xaa Xaa Xaa Xaa
               20                  25
<210> 26
   <211> 8
   <212> PRT
   <213> Artificial Sequence
<220>
   <223> synthetic peptide
<400>26
   Pro Gly Ser Pro Leu Gly Lys Asp
    1               5
<210> 27
   <211> 8
   <212> PRT
   <213> Artificial Sequence
<220>
   <223> synthetic peptide
<400> 27
   Tyr Ser Arg Leu Gly Phe Lys Asp
    1               5
<210> 28
   <211> 7
   <212> PRT
   <213> Artificial Sequence
<220>
   <223> synthetic peptide
<400> 28
   Gln Tyr Thr Gln Pro Lys Asp
   1 5
<210> 29
   <211> 8
   <212> PRT
   <213> Artificial Sequence
<220>
   <223> synthetic peptide
<400> 29
   Asn Leu Gln Gly Glu Phe Lys Asp
   1 5
<210> 30
   <211> 7
   <212> PRT
   <213> Artificial Sequence
<220>
   <223> synthetic peptide
<400> 30
   Arg Ser Phe Tyr Tyr Arg Asp
   1 5
<210> 31
   <211> 8
   <212> PRT
   <213> Artificial Sequence
<220>
   <223> synthetic peptide
<400> 31
   Ile Gln Glu Phe Gly Val Lys Asp
   1 5
<210> 32
   <211> 8
   <212> PRT
   <213> Artificial Sequence
<220>
   <223> synthetic peptide
<400> 32
   Ser Phe Arg Val Gly Tyr Lys Asp
   1 5
<210> 33
   <211> 10
   <212> PRT
   <213> Artificial Sequence
<220>
   <223> synthetic peptide
<400> 33
   Lys Asp Val Tyr Ser Leu Trp Pro Lys Asp
   1 5 10
<210> 34
   <211> 5
   <212> PRT
   <213> Artificial Sequence
<220>
   <223> synthetic peptide
<400> 34
   Ala Cys Lys Gln Phe
   1 5
<210> 35
   <211> 5
   <212> PRT
   <213> Artificial Sequence
<220>
   <223> synthetic peptide
<400> 35
   Ala Ser Lys Gln Phe
   1 5
<210> 36
   <211> 6
   <212> PRT
<220>
<400> 36
   Ala Cys Lys Arg Pro Pro
   1 5
<210> 37
   <211> 6
   <212> PRT
   <213> Artificial Sequence
<220>
   <223> synthetic peptide
<400> 37
   Ala Cys Lys Gln Phe Asn
   1 5
<210> 38
   <211> 8
   <212> PRT
   <213> Artificial Sequence
<223> synthetic peptide
<400> 38
   Ala Cys Lys Gln Phe Asp Phe Cys
   1 5
<210> 39
   <211> 9
   <212> PRT
   <213> Artificial Sequence
<220>
   <223> synthetic peptide
<400> 39
   Ala Cys Lys Gln Phe Asn Asn Asp Val
   1 5
<210> 40
   <211> 10
   <212> PRT
   <213> Artificial Sequence
<220>
   <223> synthetic peptide
<400> 40
   Ala Cys Lys Gln Phe Ala Cys Lys Gln Phe
   1 5 10
<210> 41
   <211> 11
   <212> PRT
   <213> Artificial Sequence
<220>
   <223> synthetic peptide
<400> 41
   Ala Cys Lys Gln Phe Asp Phe Cys Asn Asp His
   1 5 10
<210> 42
   <211> 12
   <212> PRT
   <213> Artificial Sequence
<220>
   <223> synthetic peptide
<400> 42
   Ala Cys Lys Arg Pro Pro Val Ser Leu Thr Cys His
   1 5 10
<210> 43
   <211> 12
   <212> PRT
   <213> Artificial Sequence
<220>
   <223> synthetic peptide
<400> 43
   Ala Cys Lys Gln Leu His Ser Val Ser Gly Cys Arg
   1 5 10
<210> 44
   <211> 12
   <212> PRT
   <213> Artificial Sequence
<220>
   <223> synthetic peptide
<400> 44
   Ala Cys Lys Gln Phe Asn Asn Asp Val Thr Cys Asn
   1 5 10
<210> 45
   <211> 14
   <212> PRT
   <213> Artificial Sequence
<220>
   <223> synthetic peptide
<400> 45
   Ala Cys Lys Gln Phe Asp Phe Cys Asn Asp His Trp Ser Lys
   1 5 10
<210> 46
   <211> 15
   <212> PRT
   <213> Artificial Sequence
<220>
   <223> synthetic peptide
<400> 46
   Ala Cys Lys Gln Phe Asn Asn Asp Val Thr Cys Asn Lys Arg His
   1 5 10 15
<210> 47
   <211> 17
   <212> PRT
   <213> Artificial Sequence
<220>
   <223> synthetic peptide
<400> 47
   Ala Cys Lys Gln Phe Asp Phe Cys Asn Asp His Trp Ser Lys Ser Gln
   1 5 10 15
   His
<210> 48
   <211> 18
   <212> PRT
   <213> Artificial Sequence
<220>
   <223> synthetic peptide
<400> 48
   Ala Cys Lys Gln Phe Asn Asn Asp Val Thr Cys Asn Ala Ala Glu Gly
   1 5 10 15
   Asp Asp
<210> 49
   <211> 12
   <212> PRT
   <213> Artificial Sequence
<220>
   <223> synthetic peptide
<400> 49
   Lys Cys His Pro Asn Met Ser Leu Lys Glu Cys His
   1 5 10
<210> 50
   <211> 12
   <212> PRT
   <213> Artificial Sequence
<220>
   <223> synthetic peptide
<400> 50
   Arg Cys Arg Arg Pro Ala Thr Thr Pro Ala Cys Arg
   1 5 10
<210> 51
   <211> 17
   <212> PRT
   <213> Artificial Sequence
<220>
   <223> synthetic peptide
<400> 51
   Arg Cys Arg Arg Pro Ala Thr Thr Pro Ala Cys Arg Ala Cys Lys Gln
   1 5 10 15
   Phe
<210> 52
   <211> 4
   <212> PRT
   <213> Artificial Sequence
<220>
   <223> synthetic peptide
<221> VARIANT
   <222> 3
   <223> Xaa=Trp, Tyr, Ser, Phe or Thr
<400> 52
   Gln Gly Xaa Phe
   1
<210> 53
   <211> 6
   <212> PRT
   <213> Artificial Sequence
<220>
   <223> synthetic peptide
<221> VARIANT
   <222> 1
   <223> Xaa=Gln, Asn, Thr, or Ser
<221> VARIANT
   <222> 4
   <223> Xaa=Trp, Tyr, Ser, Phe or Thr
<221> VARIANT
   <222> 6
   <223> Xaa=Pro, Trp or Ser
<400> 53
   Xaa Gln Gly Xaa Phe Xaa
   1 5
<210> 54
   <211> 4
   <212> PRT
   <213> Artificial Sequence
<220>
   <223> synthetic peptide
<221> VARIANT
   <222> 3
   <223> Xaa=any amino acid
<400> 54
   Gln Gly Xaa Phe
   1
<210> 55
   <211> 16
   <212> PRT
   <213> Artificial Sequence
<220>
   <223> synthetic peptide
   <221> VARIANT
   <222> (1)...(6)
   <223> Xaa=any hydrophilic, polar or charged amino acid that may or may not be present
<221> VARIANT
   <222> 9
   <223> Xaa=any amino acid
<221> VARIANT
   <222> (11)...(16)
   <223> Xaa=any hydrophilic, polar or charged amino acid that may or may not be present
<400> 55
   Xaa Xaa Xaa Xaa Xaa Xaa Gln Gly Xaa Phe Xaa Xaa Xaa Xaa Xaa Xaa
   1               5                   10                  15
<210> 56
   <211> 6
   <212> PRT
   <213> Artificial Sequence
<220>
   <223> synthetic peptide
<221> VARIANT
   <222> 1, 4, 6
   <223> Xaa=any amino acid
<400> 56
   Xaa Gln Gly Xaa Phe Xaa
   1 5
<210> 57
   <211> 18
   <212> PRT
   <213> Artificial Sequence
<220>
   <223> synthetic peptide
<221> VARIANT
   <222> (1)...(6)
   <223> Xaa=any hydrophilic, polar, or charged amino acid that may or may not be present
<221> VARIANT
   <222> 7, 10, 12
   <223> Xaa=any amino acid
<221> VARIANT
   <222> (13)...(18)
   <223> Xaa=any hydrophilic, polar, or charged amino acid that may or may not be present
<400> 57
   Xaa Xaa Xaa Xaa Xaa Xaa Xaa Gln Gly Xaa Phe Xaa Xaa Xaa Xaa Xaa
   1 5 10 15
   Xaa Xaa

## Description

### BACKGROUND OF THE INVENTION

The present invention relates generally to cell selection methods and more specifically to methods of positively selecting cells from a population.

Isolation of rare cell types to high purity is essential for the study of cell biology and has important applications in molecular medicine. The diagnosis of cell-related diseases requires methods for detection, isolation and analysis of individual cells, even if the cells are rare.

Cell-based therapies can also involve the selection of one or more specific cell phenotypes from a heterogeneous cell population such as blood or bone marrow. For example, isolation of specific cell types is useful in gene therapy applications, in which a therapeutic gene is introduced into a cell, which in turn is introduced into a patient to deliver a therapeutic gene or protein. Isolated stem cells are also used therapeutically for transplantation into an immuno-compromised patient. Stem cell transplantation is particularly useful in cancer patients who have undergone high-dose chemotherapy to compensate for tissues of the immune system that are damaged during therapy.

Cellular selection techniques generally fall into two broad categories, negative cell selection and positive cell selection. Negative cell selection involves the removal of selected cell phenotypes from a population. Positive cell selection involves the selection or isolation of a specific cell phenotype from a larger heterogeneous population.

The use of antibodies specific for a particular cell type have been applied to positive cell selection. While the use of a cell-specific antibody can be used to efficiently isolate a particular cell from a population of cells, the release of the bound cells for further use can be problematic, in particular in maintaining cell viability. Techniques for releasing cells bound to an antibody attached to a bead can include, for example, changing the pH and temperature, use of reducing agents and/or chelating agents, and the use of proteases. However, each of these techniques has disadvantages in terms of maintaining cell viability or maintaining their usefulness for therapeutic applications. For example, antibody binding to the cell surface can induce changes in the cell's physiology (Berardi et al., Science 267:104-108 (1995)).

One method useful for positively selecting cells uses an antibody specific for a particular cell and a displacing peptide (U.S. Patent No. 5,968,753, issued October 19, 1999). The method is advantageous in that cells specifically bound to the antibody can be displaced with the peptide, resulting in release of a cell that is free of bound antibody. However, such a method requires that each antibody specific for a given cell be screened for a peptide that will function to displace a pre-bound antibody-cell complex. Therefore, the method requires the identification of an antibody specific for a each cell type and screening for a peptide that can displace a cell bound to the antibody.

WO 94/0216 describes a hapten, which can be biotin or an oligopeptide conjugated to a first ligand, which is used for the isolation of cells by means of a secondary ligand specific for the hapten.

Thus, there exists a need for methods to positively select cells while maintaining cell viability and usefulness in therapeutic applications and, further, methods that can be applied universally to positively select a variety of cell types. The present invention satisfies this need and provides related advantages as well.

### SUMMARY OF THE INVENTION

In accordance with the present invention, there are provided peptide-ligand conjugates comprising a peptide conjugated to a first ligand specific for a particular cell type, wherein a free form of said peptide is capable of displacing a second ligand specific for said peptide from a preformed complex between a peptide-ligand conjugate and a second ligand specific for the peptide, wherein the peptide-specific ligand is specific for a target molecule on a cell, with the proviso that the "peptide-ligand conjugate" is not a fusion polypeptide in which the peptide is linearly linked to the ligand via the peptide bond to form a single, linear polypeptide. The peptide-ligand conjugate can be advantageously applied to a variety of cell types to positively select a particular cell. The peptide-ligand conjugate utilizes a peptide that, based on its ability to displace a second ligand specific for the peptide, can be conjugated to a variety of ligands having specificity for a variety of different cell types, thus eliminating the need to screen each cell-specific ligand for a peptide that can function to displace a pre-bound ligand-cell complex. The ligand to which a peptide is conjugated can be a molecule such as an antibody that specifically binds a target molecule, for example, a target molecule expressed on the surface of a cell such as a cell surface receptor.

The invention also provides a method of selecting a cell. The method includes the steps of contacting a cell population with a peptide-ligand conjugate of the invention, thereby forming a cell-conjugate complex; contacting the peptide-ligand conjugate with the second ligand specific for the peptide, wherein the peptide-specific ligand is specific for a target molecule on a cell; and isolating the cell-conjugate complex. The methods of the invention can further include the step of contacting the cell-conjugate complex with a free form of the peptide, thereby releasing the cell from the cell-conjugate complex. The invention further provides a method of enriching for a cell from a population using the peptide-ligand conjugate as described.

The invention further provides a method of selecting a cell subpopulation. The method includes the steps of contacting a cell population with a peptide-ligand conjugate, wherein the conjugate comprises a first ligand specific for a first cell in the population, where the first ligand is conjugated to a peptide capable of displacing a peptide-specific ligand from a preformed complex between the peptide-ligand conjugate and a second ligand specific for the peptide, thereby forming a first cell-conjugate complex; and with a second ligand specific for a second cell in the population, thereby forming a second cell-second ligand complex; contacting the peptide-ligand conjugate with the peptide-specific ligand; and isolating the first cell-conjugate complex and the second cell-second ligand complex. The invention also provides compositions of subpopulations of cells.

The invention further provides a kit for isolating a cell, comprising either a peptide capable of displacing an antibody specific for said peptide from a preformed complex between a peptide-ligand conjugate and the antibody, a cross-linking agent that allows the peptide to be conjugated to the ligand, and said antibody, wherein the antibody is specific for a target molecule on a cell, or the peptide-ligand-conjugate of any of claims 1-10 and said antibody, wherein the antibody is specific for a target molecule on a cell.

The kits of the invention can be used to couple a peptide capable of displacing an antibody specific for the peptide to any desired ligand that binds to a cell.

### BRIEF DESCRIPTION OF THE DRAWINGS

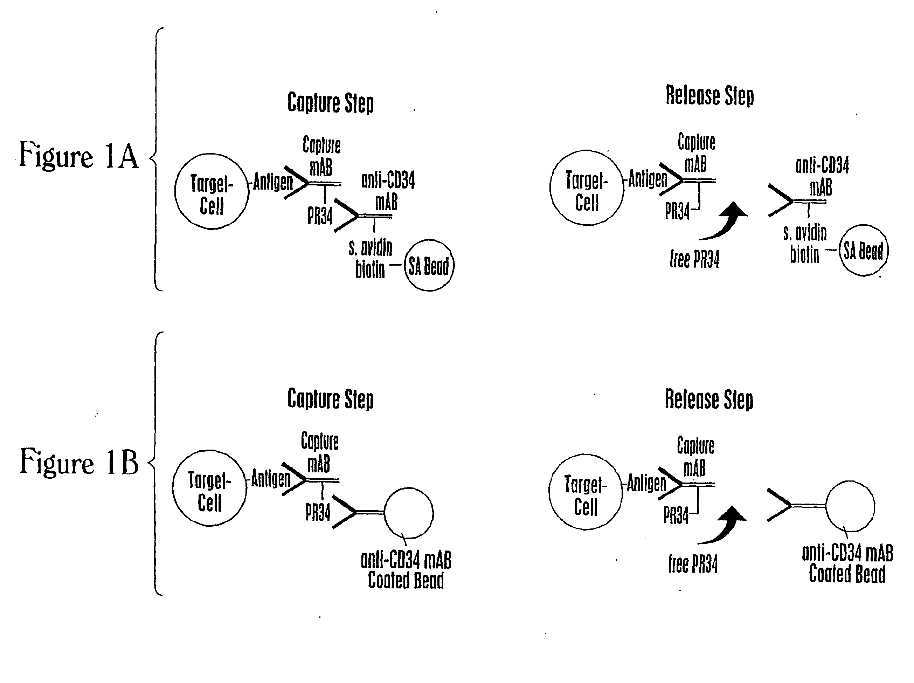

Figure 1 shows a schematic diagram of the capture and release of a target cell using a target-specific antibody conjugated to a peptide and an antibody specific for the peptide. The top panel shows capture of the peptide-antibody complex using streptavidin beads, and the bottom panel shows capture by direct binding of the peptide-specific antibody to the beads.

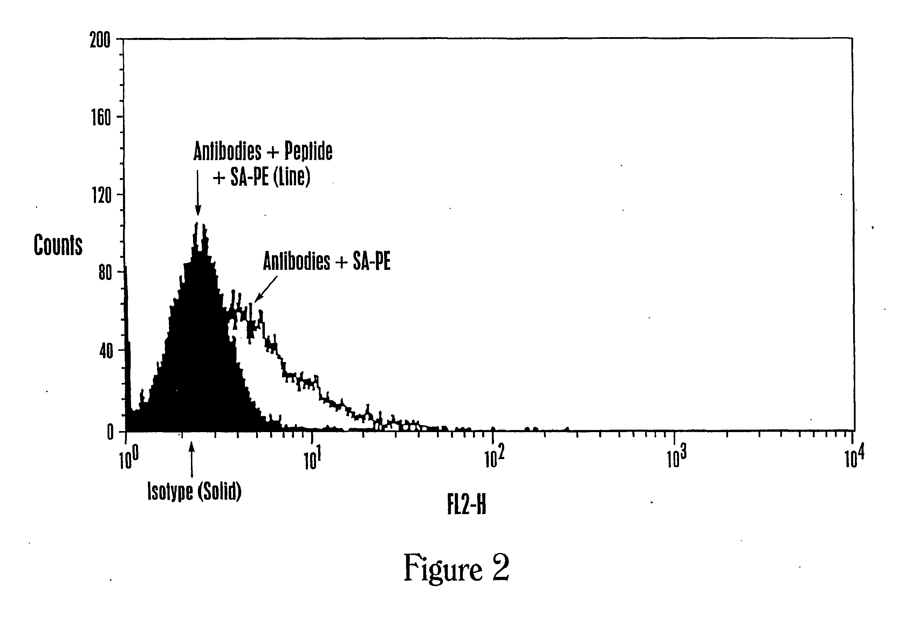

Figure 2 shows flow cytometry of SUP-T1 cells alone (Isotype), in the presence of an antibody-peptide conjugate (Antibodies + SA-PE), and in the presence 6f the antibody-peptide conjugate and free peptide (Antibodies + Peptide + SA-PE).

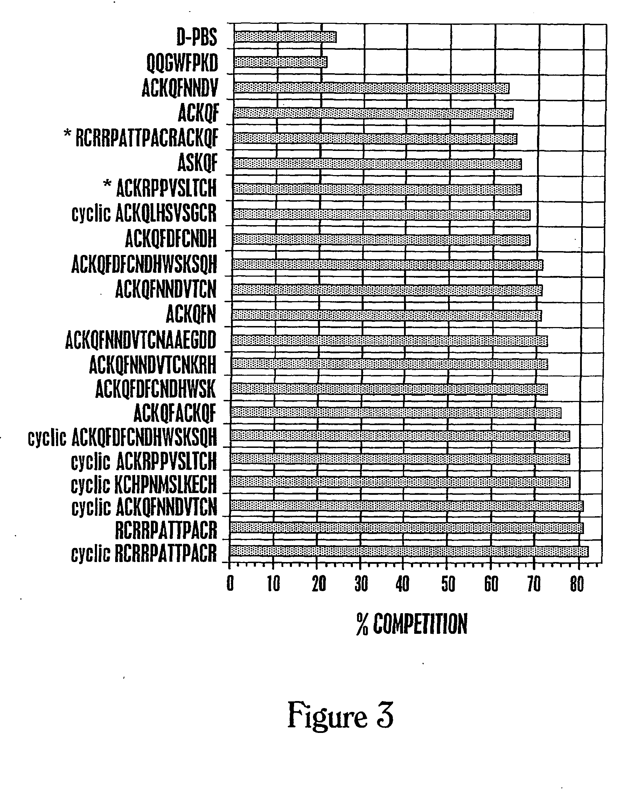

Figure 3 shows the percent competition of various peptides for an anti-CD2 antibody.

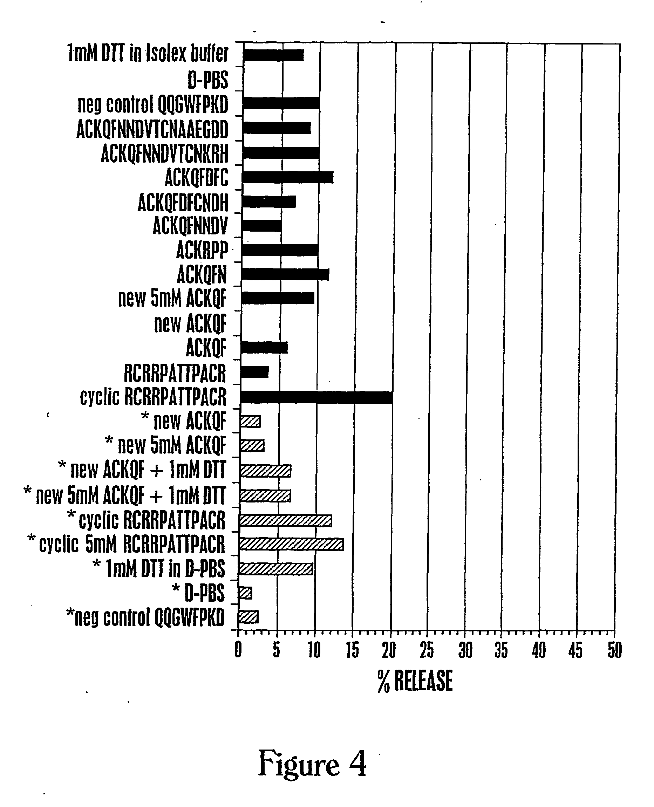

Figure 4 shows the percent release of T cells bound to anti-CD2 antibody using various peptides. The asterisk indicates data from a second assay with different donor cells.

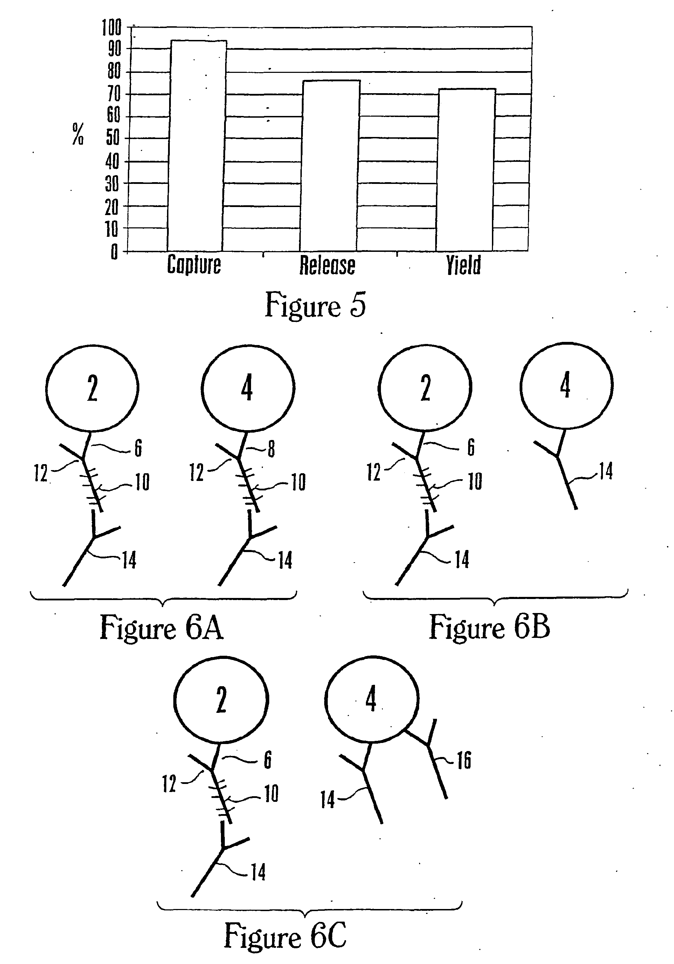

Figure 5 shows positive selection of Camma cells using Pan-O-5 monoclonal antibody and release with free peptide.
Figure 6 shows a schematic diagram of the isolation of a subpopulation of cells. Figure 6A shows the isolation of two cell types **2** and **4** with two target-specific antibody ligands (antibodies **6** and **8,** respectively). Both target-specific ligands **6** and **8** are conjugated to a peptide **10** to form a peptide-ligand conjugate **12** that is recognized by a peptide-specific antibody ligand **14.** The peptide **10** coupled to the two target-specific ligands **6** and **8** can be the same peptide or different peptides, which are capable of being displaced by a free form of the corresponding peptide **10.** Addition of free peptide(s) **10** will release cell types **2** and **4** from the peptide-specific ligand(s) **14.**
Figure 6B shows the isolation of two cell types **2** and **4.** Cell type **2** is isolated using a target-specific ligand **6** for cell type **2.** Target-specific ligand **6** is conjugated to peptide **10** to form a peptide-ligand conjugate **12.** The peptide-specific ligand **14** binds to the peptide-ligand conjugate **12.** The peptide-specific ligand **14** is also a target-specific ligand for cell type **4.** Addition of free peptide **10** will release cell types **2** and **4** from the peptide-specific ligand **14.**
Figure 6C shows the isolation of a single cell type **2** from, for example, the two cell types **2** and **4** isolated in Figure 6B, by the negative selection of a second cell type **4** so that only cell type **2** is released. Cell type **2** is bound to a target-specific ligand **6** that is conjugated to peptide **10** to form a peptide-ligand conjugate **12.** Cell type **4,** similar to Figure 6B, is bound to a peptide-specific ligand **14** that is also a target-specific ligand for cell type **4.** Cell type **4** is also bound by a second target-specific ligand **16** that is not displaced with peptide **10.** Addition of free peptide 10 will release cell type **2,** but cell type **4** remains captured by target-specific ligand **16.**

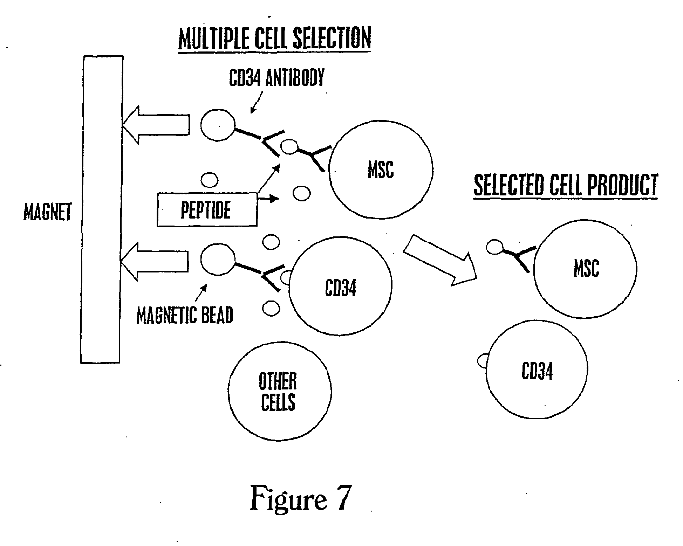

Figure 7 shows the isolation of two cell types essentially by the method shown in Figure 6B. A CD34 antibody, which can be displaced by peptide, binds directly to a CD34 cell. The CD34 antibody also binds to a mesenchymal cell (MSC)-specific ligand that is conjugated to the peptide. Both CD34 cells and mesenchymal cells are isolated away from other cells. Both CD34 and mesenchymal cells are then released from the solid support with a free form of the peptide.

### DETAILED DESCRIPTION OF THE INVENTION

In accordance with the invention, there are provided peptide-ligand conjugates comprising a peptide conjugated to a first ligand specific for a particular cell type, wherein a free form of said peptide is capable of displacing a second ligand specific for said peptide from a preformed complex between a peptide-ligand conjugate and a second ligand specific for the peptide, wherein the peptide-specific ligand is specific for a target molecule on a cell, with the proviso that the "peptide-ligand conjugate" is not a fusion polypeptide in which the peptide is linearly linked to the ligand via the peptide bond to form a single, linear polypeptide.

As used herein, the term "peptide-ligand conjugate" refers to a molecule containing at least one peptide covalently cross-linked to a ligand. A peptide-ligand conjugate retains full or partial ligand binding activity of the original ligand such that specific binding of the ligand to its binding partner is detectable. The peptide-ligand conjugate can be generated by coupling the peptide and ligand using a variety of chemical cross-linking agents as disclosed herein.

A peptide-ligand conjugate of the invention can contain multiple copies of the peptide covalently coupled to the ligand, for example, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 or more copies of the peptide. A peptide-ligand conjugate of the invention can also contain 25 or more, 30 or more, 35 or more, 40 or more, 45 or more, 50 or more, 75 or more, or even 100 or more copies of the peptide. It is understood that any number of copies of the peptide can be conjugated to the ligand so long as the ligand retains specific binding activity for its binding partner. One skilled in the art can readily optimize a peptide-ligand conjugate of the invention by coupling various amounts of peptide to the ligand and determining a number or range of peptides optimized for methods of isolating a cell from a population. As used herein, the term "peptide-ligand conjugate" specifically excludes a fusion polypeptide in which the peptide is linearly linked to the ligand via a peptide bond to form a single, linear polypeptide.

As used herein, the term "peptide" or "polypeptide" refers to a peptide or polypeptide of two or more amino acids. The term "polypeptide analog" includes any polypeptide having an amino acid sequence substantially the same as a sequence specifically described herein in which one or more residues have been conservatively substituted with a functionally similar residue and which displays the ability to displace a ligand from a parent peptide. Conservative substitutions of encoded amino acids include, for example, amino acids that belong within the following groups: (1) non-polar amino acids (Gly, Ala, Val, Leu, and Ile); (2) polar neutral amino acids (Cys, Met, Ser, Thr, Asn, and Gln) ; (3) polar acidic amino acids (Asp and Glu); (4) polar basic amino acids (Lys, Arg and His); and (5) aromatic amino acids (Phe, Trp, Tyr, and His). Other minor modifications are included within a peptide of the invention so long as the peptide retains some or all of its capability of displacing a ligand specific for the peptide.

The amino acid length of a peptide of the present invention can range from about 2 or more, about 3 or more, about 4 or more, about 5 or more, about 6 or more, about 7 or more, about 8 or more, about 9 or more, or about 10 or more amino acids. In certain embodiments, the amino acid lengths include, for example, about 11 or more, about 12 or more, about 13 or more, about 14 or more, about 15 or more, about 16 or more, about 17 or more, about 18 or more, about 19 or more, about 20 or more, about 21 or more, about 22 or more, about 23 or more, about 24 or more, about 25 or more, about 26 or more, about 27 or more, about 28 or more, about 29 or more, or about 30 or more amino acids. A peptide of the invention also includes amino acid lengths of, for example, about 32 or more, about 35 or more, about 38 or more, about 40 or more, about 45 or more, about 50 or more, about 55 or more, about 60 or more, about 65 or more, about 70 or more, about 75 or more, about 80 or more, about 90 or more, or about 100 or more amino acids.

A modification of a peptide or polypeptide can also include derivatives, analogues and functional mimetics thereof, provided that such a peptide displays the capability of displacing a ligand specific for the peptide from a preformed complex between a peptide-ligand conjugate and a second ligand specific for the peptide. For example, derivatives can include chemical modifications of the peptide such as alkylation, acylation, carbamylation, iodination, or any modification that derivatizes the peptide. Such derivatized molecules include, for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups can be derivatized to form salts, methyl and ethyl esters or other types of esters or hydrazides. Free hydroxyl groups can be derivatized to form O-acyl or O-alkyl derivatives. The imidazole nitrogen of histidine can be derivatized to form N-im-benzylhistidine. Also included as derivatives or analogues are those peptides which contain one or more naturally occurring amino acid derivatives of the twenty standard amino acids, for example, 4-hydroxyproline, 5-hydroxylysine, 3-methylhistidine, homoserine, ornithine or carboxyglutamate, and can include amino acids that are not linked by peptide bonds. Such peptide derivatives can be incorporated during synthesis of a peptide, or a peptide can be modified by well known chemical modification methods (see, for example, Glazer et al., Chemical Modification of Proteins. Selected Methods and Analytical Procedures, Elsevier Biomedical Press, New York (1975)).

Analogues of synthetic linear peptides can be made by chemically converting the structures to cyclic forms. Cyclization of linear peptides can modulate bioactivity by increasing or decreasing the potency of binding to the target protein (Pelton, J.T., et al., Proc. Natl. Acad. Sci., U.S.A., 82:236-239). Linear peptides are very flexible and tend to adopt many different conformations in solution. Cyclization acts to constrain the number of available conformations, and thus, favor the more active or inactive structures of the peptide. The immunogenicity of synthetic peptides has been correlated with the experimentally observed conformational preferences in solution (Dyson, H., et al., 1988, Annual Review of Biophysics and Biophysical Chemistry, 17:305-324). Differences in immunogenicity can be indicative of differences in binding affinity of specific antibodies for cyclic peptides.

Cyclization of linear peptides is accomplished either by forming a peptide bond between the free N-terminal and C-terminal ends (homodetic cyclopeptides) or by forming a new covalent bond between amino acid backbone and/or side chain groups located near the N- or C-terminal ends (heterodetic cyclopeptides) (Bodanszky, M., Principles of Peptide Synthesis, Springer-Verlag (1984)). The latter cyclizations use alternate chemical strategies to form covalent bonds, for example, disulfides, lactones, ethers, or thioethers. Generally, linear peptides of more than five residues can be cyclized relatively easily. The propensity of the peptide to form a beta-turn conformation in the central four residues facilitates the formation of both homo- and heterodetic cyclopeptides. The presence of proline or glycine residues at the N- or C-terminal ends also facilitates the formation of cyclopeptides, especially from linear peptides shorter than six residues in length.

A modification of a peptide of the invention includes functional mimetics thereof. Mimetics encompass chemicals containing chemical moieties that mimic the function of the peptide in displacing a ligand specific for the peptide. For example, if a peptide contains two charged chemical moieties having functional activity, a mimetic places two charged chemical moieties in a spatial orientation and constrained structure so that the charged chemical function is maintained in three-dimensional space. Thus, a mimetic, which orients functional groups that provide a function of an invention peptide to displace a ligand specific for the peptide, are included within the meaning of a peptide of the invention. All of these modifications are included within the term "peptide" or "polypeptide" so long as the peptide retains its function in displacing a ligand specific for the peptide.

As used herein, the term "ligand" refers to a molecule that can selectively bind to a molecule. The term selectively means that the binding interaction between the ligand and its binding partner is detectable over non-specific interactions by a quantifiable assay. A ligand can be essentially any type of molecule such as polypeptide, nucleic acid, carbohydrate, lipid, or any organic derived compound. Moreover, derivatives, analogues and mimetic compounds are also intended to be included within the definition of this term. In one embodiment, a ligand of the invention can be an antibody.

As used herein, the phrase "capable of displacing" when used in reference to a peptide refers to the ability of the peptide to disrupt a preformed complex of a ligand with its binding partner. The disruption of the preformed complex allows displacement of the ligand from the complex. An exemplary preformed complex is a complex between a peptide-ligand conjugate and a second ligand specific for the peptide. In such a case, a peptide capable of displacing a ligand specific for the peptide can displace a peptide-ligand conjugate prebound with a second ligand, thereby releasing the peptide-ligand conjugate from the second ligand. A ligand specific for a peptide capable of displacing the ligand can be, for example, an antibody.

As used herein, a "free form of a peptide" refers to a peptide that is not conjugated to a ligand. The free form of a peptide can be identical to a peptide that is conjugated to a ligand. Alternatively, the free form of the peptide can be a modification of the peptide conjugated to a ligand so long as the free form of the peptide has the ability to displace a second ligand specific for the peptide. In methods of the invention, the free form of the peptide is capable of displacing a preformed complex between a peptide-ligand conjugate and a second ligand specific for the peptide.

One skilled in the art can readily determine the amount of a free form of a peptide sufficient to displace a pre-formed complex between a peptide-ligand conjugate and a second ligand specific for the peptide. The free peptide displaces an amount of pre-formed complexes sufficient for use in methods of the invention, and it is understood that the free peptide need not displace all pre-formed complexes. The amount of free peptide used to displace a complex can be optimized by varying the ratio of peptide to complex to determine an amount of peptide that is optimized to displace a preformed complex. For example, a pre-formed complex in which a cell is bound to a peptide-ligand conjugate which in turn is bound to a second ligand specific for the peptide which in turn is bound to a solid support such as a bead can be tested for an optimized amount of free peptide by incubating the complex bound to the beads with various amounts of free peptide and determining an amount of free peptide that is optimized for displacement of the pre-formed complex. Thus, varying the peptide to bead ratio can be used to determine an optimized amount of free peptide to use for displacement.

A ligand specific for a peptide capable of displacing the ligand from a preformed complex between a peptide-ligand conjugate and a second ligand specific for the peptide, also referred to as a peptide-specific ligand, will generally have an affinity of about 1x10⁵ M⁻¹ to about 1x10¹³ M⁻¹ for the peptide. The peptide-specific ligand can be used to capture a target cell in a manner that allows the cell to be released by a free form of the peptide. The effectiveness of a peptide in displacing a pre-formed complex is a function of the affinity of the peptide for a ligand in the complex. Generally, such a peptide capable of displacing a ligand from a preformed complex between a peptide-ligand conjugate and a second ligand specific for the peptide will have a high on rate (kₒₙ), which allows the peptide to bind more efficiently when a pre-bound complex dissociates. Peptides particularly useful in the invention have high affinity for a ligand and are generally relatively small.

Exemplary peptides capable of displacing a ligand specific for the peptide from a preformed complex between a peptide-ligand conjugate and a second ligand specific for the peptide includes, for example, the peptides QQGWFP (SEQ ID NO:1); QQGWFPKD (SEQ ID NO:2); and QQGWFPKDC (SEQ ID NO:3), as disclosed herein. Any of the peptides capable of displacing a ligand useful in the invention can be modified to be acetylated at the amino terminus or amidated on the carboxyl terminus.

Additional exemplary peptides include, for example, peptides that displace the 9069 antibody produced by ATCC HB 11646 (see, for example, U.S. Patent No. 5,968,753). Such peptides include Q Q G F F P (SEQ ID NO:4); T Q G T F S (SEQ ID NO:5); N Q G Y F P (SEQ ID NO:6). Others include Q G X₁ F (SEQ ID NO: 52) and X₂ Q G X₁ F X₃ (SEQ ID NO:53), wherein X₁= W, Y, S, F or T; X₂= Q, N, T, or S; and X₃= P, W, or S.

Additional peptides that displace the 9069 antibody include Q G X F (SEQ ID NO:54) ; J₁ Q G X F J₂ (SEQ ID NO:55) ; X Q G X F X (SEQ ID NO:56) ; J₁ X Q G X F X J₂ (SEQ ID NO:57) ; J₁ Q Q G W F P J₂ (SEQ ID NO:7) ; J₁ T Q G S F W J₂ (SEQ ID NO:8) ; J₁ Q Q G W F P K D J₂ (SEQ ID NO:9) ; J₁ Q Q G W F P D K J₂ (SEQ I D NO:10) ; J₁ A D G A X Q G X F X G A K D J₂ (SEQ ID NO:11) ; J₁ A D G A Q Q G W F P G A K D J₂ (SEQ ID NO:12) ; J₁ A D G A T Q G S F W G A K D J₂ (SEQ ID NO:13) ; J₁ N S S V Q S J₂ (SEQ ID NO:14) ; J₁ A D G A L I S Q V S G A K D J₂ (SEQ ID NO:15) ; J₁ L I S Q V S J₂ (SEQ ID NO:16) ; J₁ N S S V X X J₂ (SEQ ID NO:17) ; J₁ N S S V G L J₂ (SEQ ID NO: 18) ; J₁ T G Q A S T J₂ (SEQ ID NO:19); J₁ A D G A P F W G Q Q G A K D J₂ (SEQ ID NO:20); J₁ A D G A T Q G T F S G A K D J₂ (SEQ ID NO:21); J₁ P E L P T Q G T F S N V S K E J₂ (SEQ ID NO:22); J₁ A D G A T Q G I C L G A K D J₂ (SEQ ID NO: 23) ; J₁ E V K L T Q G I C L E Q N K T J₂ (SEQ ID NO:24) and J₁ A D G A N Q G Y F P G A K D J₂ (SEQ ID NO:25), wherein J₁ and J₂ are selected from the group consisting of 0 - 6 amino acid residues and X is any amino acid. J¹ and J₂ contain hydrophilic, polar, or charged amino acid residues to aid the solubility of the peptide in aqueous solution. Examples of hydrophilic, polar, or charged amino acids are: G, S, T, C, Y, N, Q, D, E, H, K and R.

Exemplary peptides capable of releasing cells bound by the anti-CD34 antibody designated 9079, which is produced by the hybridoma deposited with the ATCC, designated ATCC HB-11885, include PGSPLGKD (SEQ ID NO:26); YSRLGFKD (SEQ ID NO:27); QYTQPKD (SEQ ID NO:28); NLQGEFKD (SEQ ID NO:29); RSFYYRD (SEQ ID NO:30); IQEFGVKD (SEQ ID NO:31); SFRVGYKD (SEQ ID NO:32); KDVYSLWPKD (SEQ ID NO:33).

Exemplary peptides which compete for binding of the anti-CD2 antibody B-E2 to cells include ACKQF (SEQ ID NO:34) ; ASKQF (SEQ ID NO:35) ; ACKRPP (SEQ ID NO:36) ; ACKQFN (SEQ ID NO:37) ; ACKQFDFC (SEQ ID NO:38) ; ACKQFNNDV (SEQ ID NO:39) ; ACKQFACKQF (SEQ ID NO:40) ; ACKQFDFCNDH (SEQ ID NO:41) ; ACKRPPVSLTCH (SEQ ID NO:42) ; ACKQLHSVSGCR (SEQ ID NO:43) ; ACKQFNNDVTCN (SEQ ID NO:44) ; ACKQFDFCNDHWSK (SEQ ID NO:45) ; ACKQFNNDVTCNKRH (SEQ ID NO:4,6) ; ACKQFDFCNDHWSKSQH (SEQ ID NO:47) ; ACKQFNNDVTCNAAEGDD (SEQ ID NO:48) ; KCHPNMSLKECH (SEQ ID NO:49) ; RCRRPATTPACR (SEQ ID NO:50) ; and RCRRPATTPACRACKQF (SEQ ID NO:51) (see Example VI).

Other peptides comprise an amino acid sequence selected from the group consisting of ACKQF (SEQ ID NO:34); ASKQF (SEQ ID NO:35); ACKRPP (SEQ ID NO:36); ACKQFN (SEQ ID NO:37); ACKQFDFC (SEQ ID NO:38);' ACKQFNNDV (SEQ ID NO:39); ACKQFACKQF (SEQ ID NO:40); ACKQFDFCNDH (SEQ ID NO:41); ACKRPPVSLTCH (SEQ ID NO:42); ACKQLHSVSGCR (SEQ ID NO:43); ACKQFNNDVTCN (SEQ ID NO:44); ACKQFDFCNDHWSK (SEQ ID NO:45) ; ACKQFNNDVTCNKRH (SEQ ID NO:46); ACICQFDECNDHWSKSQH (SEQ ID NO:47); ACKQFNNDVTCNAAEGDD (SEQ ID N0:43); KCHPNMSLKECH (SEQ ID NO:49) ; RCRRPATTPACR (SEQ ID NO:50); and RCRRPATTPACRACKQF (SEQ ID NO:51).

Methods for identifying a peptide capable of displacing a ligand from a pre-formed complex are well known to those skilled in the art as taught, for example, in U.S. Patent No. 5,968,753, issued October 19, 1999, and PCT publication WO 95/34817. To identify a peptide capable of displacing a ligand specific for the peptide, peptides that bind to an antibody can be initially determined. For example, a competitive binding assay can be performed to determine which peptides can prevent binding of an antibody to an antigen (see Example VI). Those peptides which are competitive for antigen binding can be further tested to determine those which can displace a performed complex between the antibody and antigen, either an isolated antigen or an antigen attached to a cell. Those peptides which displace a preformed complex can be further tested to identify any peptides having optimized displacing activity, for example, peptides that are optimized for displacing a cell from a bead. Thus, peptides can be identified having optimized displacing activity for any desired application, including displacement of a performed antibody complex with a cell or displacement of cell bound to a solid support such as a bead.

The peptides capable of displacing a ligand from a pre-formed complex can be modified, if desired, to facilitate conjugation of the peptide to a ligand. For example, as disclosed herein, a Cys residue was added to facilitate cross-linking of the peptide QQGWFPKD (SEQ ID NO:2). Methods of coupling a peptide via a Cys residue are well known to those skilled in the art including, for example, using EMCS or MBS (see Example I; Pierce, Rockford IL). It is understood that peptides can be modified by any of the well known methods to facilitate conjugation to a ligand so long as the peptide retains its ability to bind to a peptide-specific ligand.

Although the peptides can be modified to facilitate conjugation of a peptide to a ligand, the same modification need not be included in the free form of the peptide when the peptide is used to displace a second ligand specific for the peptide. Furthermore, the free form of the peptide need not have an identical amino acid sequence as the peptide conjugated to the ligand so long as the free form of the peptide is capable of displacing a second ligand bound to the peptide-ligand conjugate. Moreover, the free form of the peptide can also be a modification of the conjugated peptide, including a peptidomimetic or any polypeptide modification, as disclosed herein, so long as the free form of the peptide is capable of displacing a second ligand bound to the peptide-ligand conjugate from a preformed complex between a peptide-ligand conjugate and a second ligand specific for the peptide.

Ligands useful for conjugating to a peptide bind to a target molecule on a cell surface such as a cell surface receptor. Such a ligand, also referred to as a target-specific ligand, is useful for conjugating to a peptide and can be, for example, an antibody specific for a cell surface molecule or a ligand that binds to a cell surface molecule or receptor. A ligand can be a naturally occurring ligand or can be a modification of a naturally occurring ligand. A ligand can also be a chemical that is screened for binding activity to a target molecule on a cell surface. Methods for identifying a ligand to a cell surface molecule are well known to those skilled in the art, as disclosed herein.

As exemplary ligands, the invention provides antibodies that specifically bind to a target and function as a target-specific ligand or that are specific for a peptide capable of displacing the ligand from a preformed complex between a peptide-ligand conjugate and a second ligand specific for the peptide and function as a peptide-specific ligand. As used herein, the term "antibody" is used in its broadest sense to include polyclonal and monoclonal antibodies, as well as antigen binding fragments of such antibodies. When using polyclonal antibodies, the polyclonal sera can be affinity purified using the antigen to generate monospecific antibodies having reduced background binding and a higher concentration of antigen-specific antibodies.

An antibody of the invention, or antigen binding fragment of such an antibody, is characterized by having specific binding activity for a target molecule or a fragment thereof of at least about 1 x 10⁵ M⁻¹. Thus, Fab, F(ab')₂, Fd and Fv fragments of an invention antibody, which retain specific binding activity for a target molecule, are included within the definition of an antibody. Specific binding activity of an antibody to a target molecule can be readily determined by one skilled in the art, for example, by comparing the binding activity of an antibody to a target molecule versus a control molecule that is not the target molecule. Methods of preparing polyclonal or monoclonal antibodies are well known to those skilled in the art (see, for example, Harlow and Lane, Antibodies: A Laboratory Manual, Cold Spring Harbor Laboratory Press (1988)).

In addition, the term "antibody" as used herein includes naturally occurring antibodies as well as non-naturally occurring antibodies, including, for example, single chain antibodies, chimeric, bifunctional and humanized antibodies, as well as antigen-binding fragments thereof. Such non-naturally occurring antibodies can be constructed using solid phase peptide synthesis, can be produced recombinantly'or can be obtained, for example, by screening combinatorial libraries consisting of variable heavy chains and variable light chains as described by Huse et al. (Science 246:1275-1281 (1989)). These and other methods of making, for example, chimeric, humanized, CDR-grafted, single chain, and bifunctional antibodies are well known to those skilled in the art (Winter and Harris, Immunol. Today 14:243-246 (1993); Ward et al., Nature 341:544-546 (1989) ; Harlow and Lane, supra, 1988); Hilyard et al., Protein Engineering: A practical approach (IRL Press 1992); Borrabeck, Antibody Engineering, 2d ed. (Oxford University Press 1995)).

Antibodies can be raised using an immunogen such as an isolated target molecule, a fragment thereof, or a peptide, which can be prepared from natural sources produced recombinantly or chemically synthesized. A non-immunogenic or weakly immunogenic target molecule or portion thereof can be made immunogenic by coupling the hapten to a carrier molecule such as bovine serum albumin (BSA) or keyhole limpet hemocyanin (KLH). Various other carrier molecules and methods for coupling a hapten to a carrier molecule are well known in the art (see, for example, Harlow and Lane, *supra,* 1988). An immunogenic fragment can also be generated by expressing a portion of a target molecule that is a polypeptide as a fusion protein, for example, to glutathione S transferase (GST), polyHis or the like. Methods for expressing peptide fusions are well known to those skilled in the art (Ausubel et al., Current Protocols in Molecular Biology (Supplement 47), John Wiley & Sons, New York (1999)).

When the target-specific ligand and the peptide-specific ligand are both antibodies, the two antibodies can be from two different species. The use of two different species is particularly useful when using a secondary antibody to specifically isolate a complex via the peptide interaction (see below). For example, the target-specific antibody can be a human antibody and the peptide-specific antibody can be a mouse antibody. Exemplary species useful for generating antibodies include mammalian species such as human, mouse, rabbit, rat, sheep and goat as well as non-mammalian species such as chicken. A target-specific antibody and peptide-specific antibody can be any species so long as the target-specific and peptide-specific antibodies are from different species.

The antibodies are specific for cell surface antigens. For example, antibodies specific for cell surface markers such as CD2, CD3, CD4, CD8, CD16, CD19, CD20, CD25, CD34, CD56, and CD69 are exemplary antibodies that can be used as ligands in the invention. The cell surface markers can be specific for a particular cell type or a group of cells. For example, CD2 is a pan T cell marker for all T cells whereas CD8 is a marker for a subset of T cells. Accordingly, antibody ligands specific for these cell surface markers can be used to isolate T cells or a subset of T cells that are CD8 positive, respectively.

Other exemplary antibodies useful as ligands in the invention include antibodies specific for tumor cell markers, including HER2/neu, CD138, p53, prostate specific antigens, melanoma associated antigens (MAGE) 1, 2, 3, 4, 6, or 12, gp100, carcinoembryonic antigen (CEA), the Pan-O-5 marker and the like, and markers for tumor types such as leukemia or lymphoma, including CD19, CD20, CD22, CD25 and CD30. The Pan-O-5 antibody can bind to epithelial tumors.

Other exemplary antibodies useful as ligands in the invention include antibodies specific for stem cells. Such stem cells include, for example, a blood-generated stem cell such as a hematopoietic stem cell, a mesenchymal stem cell, a neuronal stem cell, or a muscle stem cell. Essentially, any type of all surface molecule can be used as a target for a ligand useful in the invention. Such cell surface molecules include cell surface receptors, for example, growth factor or cytokine receptors.

The peptides of the invention, which are capable of displacing a ligand specific for the peptide from a preformed complex between a peptide-ligand conjugate and a second ligand specific for the peptide, can be conjugated to any ligand specific for a desired target. The peptides of the invention can be conjugated to any target-specific ligand that is specific for a desired target such as a particular cell type (Figure 1). For example, a target-specific ligand can be a known ligand specific for a cell surface receptor on a particular cell type. A target-specific ligand can also be generated by methods well known to those skilled in the art. For example, an antibody ligand specific for a target on the surface of a cell can be generated using the antibody production methods disclosed herein by immunizing with a desired cell type or a cell type-specific target molecule and screening for antibodies that specifically bind to the desired cell type. Specific binding to a particular cell type can be determined by one skilled in the art, for example, by comparing binding of the ligand to a desired cell with binding to a control cell that does not express the cell-type specific target molecule.

In addition to using antibodies, a ligand specific for a target molecule expressed on the surface of a cell can be a naturally occurring ligand that binds to a receptor on the cell surface. Examples of such naturally occurring ligands and receptors include growth factors and cytokines and their cognate receptors. In addition to known ligand-receptor binding partners, naturally occurring ligands that bind to orphan receptors can be determined by methods well known to those skilled in the art. For example, an expression library can be screened with an orphan receptor to identify a naturally occurring ligand that binds to the receptor. Additionally, a naturally occurring ligand can be identified using screening assays such as yeast two-hybrid assays (see, for example, U.S. Patent Nos. 5,283,173, 5,468,614 and 5,667,973; Ausubel et al., *supra,* 1999; Luban et al., Curr. Opin. Biotechnol. 6:59-64 (1995)). Such naturally occurring ligands can be conjugated to a peptide capable of displacing a ligand specific for the peptide.

In the case of an antibody-expressing T cell, which expresses an antibody on the cell surface, a target-specific ligand can be an antigen specific for the antibody expressed in a T cell. The methods of the invention can thus be used to isolate or enrich for a T cell or population of T cells expressing antibodies specific for an antigen.

In addition to naturally occurring ligands, non-naturally occurring ligands specific for a target molecule can also be conjugated to a peptide of the invention. A non-naturally occurring ligand can be, for example, a derivative of a naturally occurring ligand, as described above for polypeptides. In addition, a non-naturally occurring ligand can be a compound that is screened for binding activity to a target molecule. Methods for producing pluralities of compounds to use in screening for compounds that can function.as a ligand for a target molecule, including chemical or biological molecules such as simple or complex organic molecules, metal-containing compounds, carbohydrates, peptides, proteins, peptidomimetics, glycoproteins, lipoproteins, nucleic acids, antibodies, and the like, are well known in the art and are described, for example, in Huse, U.S. Patent No. 5,264,563: Francis et al., Curr. Opin. Chem. Biol. 2:422-428 (1998); Tietze et al., Curr. Biol., 2:363-371 (1998); Sofia, Mol. Divers. 3:75-94 (1998); Eichler et al., Med. Res. Rev. 15:481-496 (1995); and the like. Libraries containing large numbers of natural and synthetic compounds also can be obtained from commercial sources. Combinatorial libraries of molecules can be prepared using well known combinatorial chemistry methods (Gordon et al., J. Med. Chem. 37: 1233-1251 (1994); Gordon et al., J. Med. Chem. 37: 1385-1401 (1994); Gordon et al., Acc. Chem. Res. 29:144-154 (1996); Wilson and Czarnik, eds., Combinatorial Chemistry: Synthesis and Application, John Wiley & Sons, New York (1997)).

Any peptide-ligand binding pair in which the peptide is capable of displacing the ligand specific for the peptide from a preformed complex between a peptide-ligand conjugate and a second ligand specific for the peptide, wherein the peptide-specific ligand is specific for a target molecule on a cell can be used in combination in methods of the invention for isolating a cell. The peptide can be conjugated to any target-specific ligand to allow isolation of a target cell by the peptide-ligand conjugate. The cell can then be displaced from the preformed complex with a free form of the peptide.

In one embodiment, a ligand specific for a peptide capable of displacing the ligand specifically binds CD34. One such ligand that specifically binds CD34 is monoclonal antibody 9C5. The anti-CD34 antibody 9C5, also known as antibody 9069, was deposited in the American Type Culture Collection, 10801 University Boulevard, Manassas VA 90110, on June 7, 1994, and designated HB 11646. An example of a peptide capable of displacing the 9C5 ligand is the peptide QQGWFP (SEQ ID NO:1), QQGWFPKD (SEQ ID NO:2); and QQGWFPKDC (SEQ ID NO:3) (see Examples I to III).

A peptide-ligand conjugate can be generated, for example, by chemically cross-linking at least one copy of a peptide to a ligand specific for a target molecule. Suitable cross-linking agents include, for example, the heterobifunctional cross-linking agent N-(ε-maleimidocaproyloxy)succinimide ester (EMCS) (see Example I). Other suitable cross-linking agents include, for example, glutaraldehyde; m-maleimidobenzoyl-N-hydroxysuccinimide ester (MBS); carbodimide; bis-diazo benzidine (BDB); azidobenzoyl hydrazide (ABH); N-5-azido-2-nitrobenzoyloxysuccinimide (ANB-NOS); N-(4-(p-azidosalicylajido)butyl)-3'-(2'-pyridyldithio)propionamide (APDP); p-azidophenyl glyoxal monohydrate (APG); 4-(p-azidosalicylamido)butylamine (ASBA); 1-(p-azidosalicylamido)-4-(iodoacetamido)butane (ASIB); bis-(β-(4-azidosalicylamido)ethyl)disulfide (BASED); bismaleimidohexane (BMH); bis(sulfosuccinimidyl)suberate (BS³); bis(2-succinimidooxycarbonyloxy)ethyl)sulfone (BSOCOES); bis(2-sulfosuccinimidooxycarbonyloxy)ethyl)sulfone (sulfo-BSOCOES); 1,5-difluoro-2,4-dinitrobenzene (DFDNB); dimethyl adipimidate·2 HCl (DMA); dimethyl pimelimidate·2 HCl (DMP); dimethyl suberimidate·2 HCl (DMS); 1,4-di-(3'-(2'-pyridyldithio)-propionamido))butane (DPDPB); disuccinimidyl glutarate (DSG); dithiobis(succinimidylpropionate) (DSP); disuccinimidyl suberate (DSS); disuccinimidyl tartarate (DST); dimethyl 3,3'-dithiobispropionimidate 2 HCl (DTBP); 3,3'-dithiobis(sulfosuccinimidylproprionate) (DTSSP); 1-ethyl-3-(3-dimethylaminopropyl)-carbodiimide hydrochloride (EDC); ethylene glycolbis-(succinimidylsuccinate) (EGS); ethylene glycolbis-(sulfosuccinimidylsuccinate) (sulfo-EGS); N-γ-maleimidobutyryloxy-succinimide ester (GMBS); N-γ-maleimidobutyryloxy-sulfosuccinimide ester (sulfo-GMBS); N-hydroxysuccinimidyl-4-azidobenzoate (HSAB); -hydroxysulfosuccinimidyl-4-azidobenzoate (sulfo-HSAB); m-maleimidobenzoyl-N-hydroxysulfosuccinimide ester (sulfo-MBS); 4-(N-maleimidomethyl)-cyclohexane-I-carboxylhydrazide-HCl (M₂C₂H) ; 4- (4-N-maleimidophenyl) - butyric acid hydrazide-HCl (MPBH); N-hydroxysuccinimidyl-4-azidosalicylic acid (NHS-ASA); N-hydroxysulfosuccinimidyl-4-azidosalicylic acid (sulf-NHS-ASA); sulfosuccinimidyl(4-azidosalicylamido)hexanoate (sulfo-NHS-LC-ASA); 3-(2-pyridyldithio)propionyl hydrazide (PDPH); p-nitrophenyl-2-diazo-3,3,3,-trifluoropropionate (PNP-DTP); 2-diazo-3,3,3-trifluoropropionylchloride (DTP); N-succinimidyl(4-azidophenyl)1,3'-dithiopropionate (SADP) ; sulfosuccinimidyl(4-azidophenyldithio)propionate (sulfo-SADP) ; sulfosuccinimidyl 2-(7-azido-4-methycoumarin-3-acetamide)ethyl-1,3'-dithiopropionate (SAED) ; sulfosuccinimidyl 2-(m-azido-o-nitrobenzamido)-ethyl-1,3'-dithiopropionate (SAND); N-succinimidyl-6-(4'-azido-2'-nitrophenylamino)hexanoate (SANPAH) ; sulfosuccinimidyl-6-(4'-azido-2'-nitrophenylamino)hexanoate (sulfo-SANPAH); sulfosuccinimidyl-2-(p-azidosalicylamido)ethyl-1,3'-dithiopropionate (SASD); -hydroxysuccinimidyl-2,3-dibromopropionate (SDBP); N-succinimidyl(4-iodoacetyl)aminobenzoate (SIAB); suflosuccinimidyl(4-iodoacetyl)aminobenzoate (sulfo-SIAB); succinimidyl 4-(N-maleimidomethyl)cyclohexane-1-carboxylate (SMCC); sulfosuccinimidyl 4-(N-maleimidomethyl)cyclohexane-1-carboxylate (sulfo-SMCC); succinimidyl 4-(p-maleimidophenyl)butyrate (SMPB); sulfosuccinimidyl 4-(p-maleimidophenyl)butyrate (sulfo-SMPB); 4-succinimidyloxycarbonyl-methyl-α-(2-pyridyldithio)toluene (SMPT); sulfosuccinimidyl 6-(α-methyl-a-(2-pyridyldithio)toluamido)hexanoate (sulfo-SMPT);N-succinimidyl-3-(2-pyridyldithio)propionate (SPDP); succinimidyl 6-(3-(2-pyridyldithio)propionamido)hexanoate (LC-SPDP); sulfosuccinimidyl 6-(3-(2-pyridyldithio)propionamido)hexanoate (sulfo-LC-SPDP); sulfosuccinimidyl 7-azido-4-methylcoumarin-3-acetate (sulfo-SAMCA); sulfosuccinimidyl 4-(p-azidophenyl)butyrate (sulfo-SAPB); *N*-(α-maleimidoacetoxy)succinimide ester (AMAS); 1,8-*bis-*maleimidotriethyleneglycol (BM(PEO)₃); 1,11*-bis-*maleimidotetraethyleneglycol (BM(PEO)₄); 1,4-*bis-*maleimidobutane (BMD); 1,4-*bis*-maleimidyl-2,3-dihydroxybutane (BMDB); *N*-(β-maleimidopropionic acid)hydrazide·trifluoroacetic acid (BMPH); *N*-(β-maleimidopropyloxy)-succinimide ester (BMPS) ; *N*-ε-maleimidocaproic acid (EMCA); *N*-(ε-maleimidocaproic acid)hydrazide (EMCH); *N*-κ-maleimidoundecanoic acid (KMUA); *N*-(κ-maleimidoundecanoic acid)-hydrazide (KMUH); succinimidyl-4-(*N*-maleimidomethyl)-cyclohexane-1-carboxy(6-amidocaproate) (LC-SMCC); succinimidyl-6-((β-maleimido-propionamido)hexanoate) (SMPH); *N*-(ε-maleimidocaproyloxy)sulfosuccinimide ester (Sulfo-EMCS); *N*-(κ-maleimidoundecanoyloxy))-sulfosuccinimide ester (Sulfo-KMUS); *N*-succinimidyl-(4-vinylsulfonyl)benzoate (SVSB) (see Harlow and Lane, Antibodies: A Laboratory Manual (Cold Spring Harbor Laboratory Press (1988); Pierce Chemical Co.; Rockford IL).

The peptide-ligand conjugates of the invention and ligands specific for the peptide are particularly useful in positive cell selection techniques. A peptide capable of displacing a ligand specific for the peptide from a preformed complex between a peptide-ligand conjugate and a second ligand specific for the peptide can be coupled to any ligand that specifically binds to a cell surface target molecule and used to isolate a cell that expresses the cell surface target molecule. Exemplary peptide-ligand conjugates include the PR34^{+™} peptide (QQGWFPKDC; SEQ ID NO:3) conjugated to an anti-CD8 antibody and the PR34^{+™} peptide coupled to an anti-CD2 antibody (see Examples I and IV).

The invention also provides a method of selecting a cell. The method includes the steps of contacting a cell population with the peptide-ligand conjugate of the invention, thereby forming a cell-conjugate complex; contacting the peptide-ligand conjugate with the second ligand specific for the peptide wherein the peptide-specific ligand is specific for a target molecule on a cell; and isolating the cell-conjugate complex. A method of the invention can further comprise the step of contacting the cell-conjugate complex with a free form of the peptide, thereby releasing the cell from the cell-conjugate complex.

The methods of the invention are useful for isolating a cell from a population of cells containing various cell types. The methods of the invention use a peptide-ligand conjugate in which the ligand can specifically bind to a target cell in a population, I thereby allowing selection of the target cell from the population.

The methods of the invention are particularly useful for isolating a stem cell. Isolated stem cells can be used in various therapeutic methods, for example, in methods of expressing a therapeutic agent or in transplantation. Stem cells can be transduced with genetic elements capable of expressing a therapeutic gene product and introduced into a patient for gene therapy methods. A stem cell is particularly useful for transplantation into a patient in need of stem cell transplantation, for example, a cancer patient treated with high dose chemotherapy. Methods for processing bone marrow and stem cells, particularly for transplantation, are well known to those skilled in the art (see, for example, Areman et al., Bone Marrow and Stem Cell Processing: A Manual of Current Techniques F.A. Davis Company, Philadelphia (1992). A stem cell can be, for example, a CD34+ cell.

The methods of the invention can be used to isolate stem cells of various tissue types. The methods of the invention can be used, for example, to isolate a blood-generated stem cell, a mesenchymal stem cell, a neuronal stem cell, or a muscle stem cell. As used herein, a "blood-generated cell" refers to a cell derived from a blood cell. A blood cell is generally derived from bone marrow. Blood cells include, for example, red blood cells, mononuclear cells, leukocytes, dendritic cells, macrophages, natural killer (NK) cells, naive or memory T cells, antigen-specific T cells and the like. Although a blood-generated cell is derived from a blood cell, it is understood that the blood cell need not reside in blood. Thus, cells that are transported via blood, but migrate out of blood into various tissues are included within the meaning of a blood-generated cell. A blood-generated cell includes cells that exist or migrate via the blood system, cells derived from bone marrow, cells from the lymphatic system, or cells from cord blood. Such cells include many cells of the immune system. A blood-generated stem cell can include a hematopoietic stem cell.

The various stem cells can differentiate into various cells specific for their respective systems. Thus, neuronal stem cells can differentiate into various cells of the nervous system and mesenchymal stem cells can develop into various cells or tissue of mesenchymal origin, including musculo-skeletal, vascular and urogenital systems and connective tissue. Blood-generated stem cells such as hematopoietic stem cells can differentiate into various blood cell types such as leukocytes and their various differentiated forms, including B-cells and T-cells.

The methods of the invention are also useful for isolating cells such as T cells and T cell subsets, B cells and B cell subsets, dendritic cells, NK cells, naive or memory T cells, mesenchymal stem cells, neuronal stem cells, antigen-specific T cells, monocytes and the like. T cell subsets include T cells expressing specific markers such as CD2, CD4, CD8, CD3, CD16, CD56, CD34, CD25, and CD69. B cell subsets include B cells expressing markers such as CD19 and CD20.

The methods of the invention can also be used to isolate a tumor cell, for example, a tumor cell from tumors of various tissues including breast, prostate, colon, lung, ovary, pancreas, adrenal, and from cancers such as leukemia, myeloma, melanoma, lymphoma, renal cell carcinoma, adrenal cell carcinoma, pancreatic carcinoma or other tumor cell type. The isolation of tumor cells can be particularly useful in diagnostic methods, as described below. The methods of the invention can also be used to isolate anti-idiotype, for example, myeloma or B cell lymphoma cells where the idiotype is expressed on the cell surface. In this case, anti-idiotypic antibody conjugated to peptide recognized by a peptide-specific ligand can be used to capture a target cell. Subsequent release of the cell can be achieved by the addition of the free form of the peptide to which the peptide-specific antibody binds.

The isolation of tumor cells can also be used as a source of antigen for therapeutic methods such as a tumor vaccine. For example, a tumor cell isolated by methods of the invention can be exposed to dendritic cells *ex vivo* to antigen-prime the dendritic cells. The primed dendritic cells can then be administered to an individual to stimulate a T cell response against the tumor antigens expressed in the dendritic cells. Alternatively, the isolated tumor cells can be irradiated or rendered unable to grow by other methods and reintroduced into the individual to stimulate an immune response to tumor cell antigens expressed in the tumor cell. If desired, the isolated tumor cells can be cultured in vitro prior to exposure to dendritic cells or radiation.

As used herein, the term "specific binding" or "specific for," when used in reference to a binding interaction, means binding that is measurably different from a non-specific interaction. Specific binding can be measured, for example, by determining binding to a target molecule compared to binding to a control molecule, which generally is a molecule of similar structure that does not have binding activity. In this case, specific binding is indicated if the molecule has measurably higher affinity for the target molecule than the control molecule. Similarly, specific binding to a target cell can be determined by comparing binding to a control cell, which generally is a cell that does not express a target molecule specific to the target cell.

As used herein, selective binding refers to a binding interaction that is both specific and discriminating between molecules, for example, a ligand such as an antibody that binds to a single molecule or closely related molecules. For example, an antibody can exhibit specificity for an antigen that can be both specific and selective for the antigen if the antigenic epitope is unique to a molecule. Thus, a molecule having selective binding can differentiate between molecules, as exemplified by an antibody having specificity for an epitope unique to one molecule or closely related molecules. Alternatively, an antibody can have specificity for an epitope that is common to many molecules. Such an antibody has specific binding but is not selective for one molecule or closely related molecules.

Specificity of binding can be determined, for example, by competition with a control molecule that is known to bind to a target. For example, specific binding of a ligand to a target can be demonstrated by competing for binding with the same ligand. In this case, specific binding is indicated if the binding of a molecule is competitively inhibited by the same ligand.

The term "specific binding," as used herein, includes both low and high affinity specific binding. Specific binding can be exhibited, for example, by a low affinity interaction having a Kd of at least about 10⁻⁴ M. Specific binding also can be exhibited by a high affinity binding molecule, for example, a binding molecule having a Kd of at least about of 10⁻⁷ M, at least about 10⁻⁸ M, at least about 10⁻⁹ M, at least about 10⁻¹⁰ M, or can have a Kd of at least about 10⁻¹¹ M or 10⁻¹² M or greater. Both low and high affinity binding molecules are useful in methods of the invention.

Selective binding refers to binding of a particular ligand relatively specifically to a target molecule. In general, selective binding is characterized, in part, by detecting at least a two-fold (2x) greater specific binding of the ligand to the target molecule or target cell as compared to a control molecule or cell. Accordingly, a molecule exhibiting selective binding to a target molecule or cell has detectably distinct binding activity compared to at least one other molecule or cell.

When using methods of the invention to isolate a cell from a population, it is generally advantageous to isolate the cells on a solid support. As used herein, the term "solid support" refers to a solid medium which is sufficiently stable so as to allow immobilization of a molecule useful for isolating a cell from a population. Solid supports can include, for example, membranes such as nitrocellulose, nylon, polyvinylidene difluoride, plastic, glass, polyacrylamide, agarose or polystyrene. Solid supports can also be made in essentially any size or shape so long as it supports the immobilization of a molecule useful for isolating a cell from a population. For example, the solid support can be a flat planar surface such as a natural or synthetic membrane filter or a glass slide. Alternatively, the solid support can be of various spherical shapes, including, for example, beads made of glass, polyacrylamide or agarose. The solid support can also be a particle or ferrous solutions. Porous mediums can similarly be used as solid supports and such mediums are included within the definition of the term as used herein. Additionally, any of the solid supports can be modified, for example, to include functional chemical groups that can be used directly or indirectly for attachment of binding molecules or linkers.

The solid support can additionally exhibit properties useful in isolating a target cell. For example, the solid support can be magnetic microspheres, which are useful in isolating cells from a population (see Hardwick et al., J. Hematotherapy 1:379-386 (1992); Egeland et al., Scand. J. Immunol. 27:439-444 (1988); Dynal, Lake Success NY).

For isolation of a cell from a population, the ligand specific for the peptide in a peptide-ligand conjugate can be attached to a solid support. The peptide-specific ligand can be covalently coupled to the solid support or attached to the support via non-covalent interactions. For example, the peptide-specific ligand can be directly bound to beads (see Figure 1 and Example V). For direct coupling of the ligand to the solid support, cross-linking agents, as disclosed herein, can be used to covalently couple the ligand to the solid support. For attachment of the ligand to a support via non-covalent interactions, the ligand can be directly attached to the support and bound by non-covalent interactions, for example, by directly binding the ligand to beads (see Figure 1 and Example V). The direct binding of a ligand to beads or other solid support is mediated by biochemical and/or biophysical properties of the beads, such as hydrophilicity, hydrophobicity, ionic character, and the like. Alternatively, the ligand can be attached to the support via an intermediary interaction, for example, a secondary antibody in the case of an antibody ligand. A molecule having binding activity for the ligand such as a secondary antibody or reagent can be attached to the solid support, generally via covalent coupling to the solid support. The ligand is then attached to the solid support via non-covalent interactions with the ligand-specific molecule covalently attached to the solid support.

In the case where the ligand is an antibody, the ligand-specific molecule attached to the solid support can be a secondary antibody specific for the antibody. For example, if the antibody is a mouse monoclonal antibody, the secondary antibody attached to the solid support can be an anti-mouse heavy and/or light chain antibody or an anti-isotype antibody. When selecting a secondary antibody, the antibody is preferably specific for the peptide-specific ligand and not the target-specific ligand. Accordingly, when using antibodies as ligands, the peptide-specific antibody can be from a different species than the target-specific antibody. Using different species allows the convenient use of a secondary antibody that is specific for the peptide-specific antibody but does not bind the target-specific antibody. Exemplary species useful as secondary antibodies include mammalian species such as human, rabbit, mouse, rat, sheep and goat, as well as chicken and the like. The use of different species of antibody ligand allows the use of a secondary antibody specific for the peptide-specific antibody. Thus, only the cells which are bound via the peptide and peptide specific ligand interaction will be isolated by the beads, allowing displacement of the complex with free peptide.

In addition, the ligand can be modified to incorporate a moiety that is recognized by a molecule that can be bound to the solid support. For example, as disclosed herein, the ligand can be conjugated to biotin (see Examples I to III). The biotinylated ligand can then be bound to avidin or streptavidin attached to a solid support (see Figure 1). The use of biotin and avidin is particularly useful for immunoselection of cells since this is a high affinity interaction (see, for example, U.S. Patent Nos. 5,225,353, issued July 6, 1993, and 5,215,927, issued June 1, 1993). As disclosed herein, isolation of CD34+ and CD2+ cells using a peptide-ligand conjugate specific for CD34+ and CD2+ target cells, respectively, was highly efficient when isolated with streptavidin beads (see Examples III and IV).

In addition to biotin, any molecule that has an appropriate binding partner can be used to couple to a ligand. Covalent coupling can be performed using an appropriate cross-linking agent, such as those disclosed herein. Alternatively, if the ligand is expressed recombinantly, an appropriate tag having specificity for a molecule attached to the solid support can be expressed as a fusion polypeptide with the ligand. Expression of fusion polypeptides are well known to those skilled in the art (see Ausubel et al., *supra*, 1999).

When isolating a cell from a population, the efficiency of cell binding can vary depending on the ratio of cells to beads or other solid support or surface area of the solid support. Therefore, one skilled in the art can determine an optimized ratio of cells to beads or other solid support to isolate cells with the highest efficiency by varying the ratio of cells to beads and determining one that provides an optimized efficiency of cell binding and isolation.

When forming a binding complex for isolation of a target cell, the target cells, peptide-ligand conjugate, peptide-specific ligand and solid support such as beads can be combined in any order so long as the binding complex formed allows isolation of the target cell. Any of the components can be pre-incubated to form a complex prior to the combination of all components. For example, as disclosed herein, sequential incubation with target cells, peptide-ligand conjugate, peptide-specific ligand and capture beads resulted in more efficient binding of cells than when the peptide-specific ligand was in a pre-formed complex with the capture beads (see Example II). One skilled in the art can readily determine an optimized condition for incubation of components, time of incubation and ratio of beads to cells to determine optimized conditions for isolation of a particular cell using a particular peptide-ligand conjugate and peptide-specific ligand. In addition to optimizing conditions for binding of cells, the cell to bead ratio can also be optimized for displacement of cells by free peptide using various amounts of free peptide, as described above.

The invention additionally provides a method of enriching for a cell from a cell population. The method includes the steps of contacting a cell population with the peptide-ligand conjugate of the invention, thereby forming a cell-conjugate complex; contacting the peptide-ligand conjugate with the second ligand; and isolating the cell-conjugate complex. The method of the invention can further comprise the step of contacting the cell-conjugate complex with the peptide, thereby releasing the cell from the cell-conjugate complex. Furthermore, as described in more detail below, the invention provides methods of enriching for a subpopulation of cells. As used herein, enriching for a cell or subpopulation from a cell population means that the target cell(s) is at a higher concentration relative to other cells than in the original cell population. One skilled in the art can readily determine the relative purity of an enriched cell or cell population using well known immunological or histochemistry methods, as disclosed herein.

Additionally disclosed is a method of diagnosing a condition. The method includes the steps of contacting a sample comprising a cell population with the peptide-ligand conjugate of the invention, thereby forming a cell-conjugate complex; contacting the peptide-ligand conjugate with the second ligand; isolating the cell-conjugate complex; and identifying the cell in the complex, wherein the cell is associated with a condition. The method of the invention can further comprise the step of contacting the cell-conjugate complex with the peptide, thereby releasing the cell from the cell-conjugate complex.

Methods for diagnosing a condition are particularly useful for diagnosing a condition in which there are a limited number of cells associated with the condition. A sample containing a population of cells is contacted with a peptide-ligand conjugate in which the ligand is specific for a target cell associated with a condition. The sample can be, for example, a blood sample or biopsy. In the case of a tissue biopsy, the cells can be dispersed using well known methods of proteolytic digestion for obtaining primary cultures (see, for example, Jaffee et al., Cancer J. Sci. Am., 4:194-203 (1998)). In the case of a tissue biopsy, the presence of a particular cell in the dispersed cell population is determined. Dispersal of cells from a tumor sample such as a tumor biopsy can be useful for tumor vaccine methods, as disclosed herein.

One limitation of early detection methods for diseases such as cancer is that, at early stages, there are a limited number of cancer cells, which therefore can be difficult to detect. The methods of the invention can be advantageously used to concentrate the limited number of cancer cells in early stage cancer, thereby allowing the cells to be more readily detected. For example, the methods of the invention can be used as a screening method to detect early stages of cancer such as early stages of breast cancer or prostate cancer. The methods of the invention can also be applied to a sample to detect metastatic cancer cells, which can be at low concentrations in a blood sample and therefore at insufficient concentrations to allow detection using standard methods. The methods of the invention allow concentration of cells prior to the application of detection methods. Thus, the methods of the invention are useful for enriching for tumor cells for diagnostic applications, for example, enriching for tumor cells from blood or bone marrow.

Methods of identifying a particular cell type isolated or enriched from a cell population are well known to those skilled in the art, including, for example, immunological procedures, histochemical methods, or analysis of tissue-specific gene expression using polymerase chain reaction (PCR). Immunological procedures useful for detection of a particular cell type include immunoassays that employ a detectable antibody. Such immunoassays include, for example, immunohistochemistry, immunofluorescence, ELISA assays, radioimmunoassay, fluorescence activated cell sorting (FACS) analysis, immunoprecipitation, immunoblot analysis, Pandex microfluorimetric assay, agglutination assays, flow cytometry and serum diagnostic assays, which are well known in the art (Harlow and Lane, supra, 1988; Harlow and Lane, Using Antibodies: A Laboratory Manual, Cold Spring Harbor Press (1999)). Methods for identifying a cell by histochemical or immunohistochemical methods are well known to those skilled in the art (see, for example, Sumner, Basic Histochemistry Wiley-Interscience, New York (1988); Gu, ed., Analytical-Morphology: Theory, Applications, and Protocols Birkhauser Boston, Cambridge MA (1997)).

Additionally, methods for identifying a cell by PCR are well known in the art (see, for example, Dieffenbach and Dveksler, PCR Primer: A Laboratory Manual, Cold Spring Harbor Press (1995); Ausubel et al., supra, 1999). The detection of cell-specific messenger RNA expression can be performed in a single cell or a small number of cells using reverse transcriptase PCR (RT-PCR) and detection of corresponding PCR products. PCR primers can be selected to hybridize and prime an amplification reaction to detect expression of a cell-specific mRNA. Particularly useful primers for detection of a cell specific mRNA are primers that prime in regions encoded by two different exons. Such primers can be selected to allow detection of mRNA by efficient production of a PCR product of the appropriate size. In contrast, the primers which hybridize in regions encoded by two different exons would not generate a detectable product from genomic DNA due to the intervening intron sequence, which would place the primers too far apart to generate any detectable PCR product. Such primers selected to prime from two different exons are useful to detect cell-specific mRNA expression.

In some applications, it can be desirable to release a cell from the bound peptide-ligand conjugate. For example, antibody binding to cell surface antigens can induce alterations in cell physiology (Berardi et al., supra, 1995). Therefore, in applications using cells isolated by the methods of the invention in which the physiological state of the cell is potentially important, for example, in therapeutic applications, it can be desirable to displace the peptide-ligand conjugate from the isolated cells. Additionally, it can be desirable to release a cell from the peptide bound to a solid support in certain cell diagnostic techniques, for example, histochemistry and immunohistochemistry methods.

One method of displacing the peptide-ligand conjugate from a cell is to use a cell-specific ligand that itself can be displaced by a known molecule. For example, the target-specific ligand in the conjugate can be displaced by a peptide that is known, or such a peptide can be readily determined by one skilled in the art, to be capable of displacing the ligand from the cell, as disclosed herein (see U.S. Patent No. 5,968,753). In such a case, both the target-specific ligand in the peptide-ligand conjugate and the peptide-specific ligand can be displaced by a corresponding peptide.

In addition to the use of a peptide to displace the peptide-ligand conjugate from a cell, other molecules can be used in conjunction with the peptide to enhance displacement by the peptide. One skilled in the art can readily determine other reagents or components that facilitate the displacement activity of a peptide by screening for peptide displacement activity by the methods disclosed herein in the presence of various compounds. For example, the pH of the buffer, the salt concentration, or the temperature can be varied to enhance displacement with a peptide. Other agents can be added, for example, reducing agents such as dithiothreitol and/or chelating agents such as ethylenediamine tetraacetic acid (EDTA) or ethylene glycol bis(β-aminoethyl ether)-N,N,N*'*,N*'*-tetraacetic acid (EGTA) can be added to enhance displacement with a peptide.

Molecules that can function to displace the peptide-ligand conjugate from the cell can be readily determined by one skilled in the art. For example, peptides that are specific for the ligand and capable of displacing a peptide-ligand conjugate from a cell can be screened and identified using well known methods (see U.S. Patent No. 5,968,753). In addition, compounds for displacing a peptide-ligand conjugate from a cell can be identified using well known screening methods to identify a compound having displacement activity, as described above for identifying non-natural ligands for a target molecule.

The compounds can be screened, for example, to detect displacement of a target-specific ligand from the target molecule. In such screening methods, the target molecule can be expressed on the surface of the cell or expressed recombinantly. One skilled in the art will know or can readily determine methods to detect the displacement of the ligand from a pre-formed complex between the ligand and the target molecule. For screening methods, the ligand can be detectably labeled, for example, with a radiolabel, a fluorochrome, a ferromagnetic substance, a luminescent tag or a detectable binding agent such as biotin. A labeled ligand allows a compound having displacing activity for the ligand to be more readily determined.

In addition, a cell can be displaced from a complex attached to a solid support using proteolytic digestion, for example, with chymopapain, pepsin, papain, chymotrypsin and the like. Methods for releasing cells from an affinity matrix using proteases are well known to those skilled in the art (U.S. Patent No. 5,081,030, issued January 14, 1992).

The invention further provides a method of selecting a cell subpopulation. The method includes the steps of contacting a cell population both with the peotide-ligand conjugate, of the invention thereby forming a first cell-conjugate complex; and further contacting the cell population simultaneously or sequentially with a second ligand specific for a second cell in the population, thereby forming a second cell-second ligand complex; contacting the peptide-ligand conjugate with the peptide-specific ligand; and isolating the first cell-conjugate complex and the second cell-second ligand complex.

Thus, the methods of the invention can be advantageously used to isolate a single cell type from a population of cells or a subpopulation of two or more different cell types. The methods utilize one or any combination of two or more target-specific ligands specific for different cells. The cells can be isolated, for example, by binding the target-specific ligands to a solid support using the methods disclosed herein. Furthermore, the cells can be released from the solid support, for example, using a peptide, as described below.

In one embodiment, when isolating a subpopulation of cells, the subpopulation of cells can be conveniently released using the same peptide capable of displacing the target-specific ligand (see Figure 6). For example, if the peptide-specific ligand (e.g., the 9069 antibody and the like) is also specific for a target molecule on a cell (e.g., CD34 antigen and the like), the peptide-specific ligand can be simultaneously used to isolate a cell bound to a corresponding peptide-ligand conjugate (Figure 6B, cell type 2) and the target cell to which the peptide-specific ligand binds (Figure 6B, cell type 4), herein the target molecule (e.g., CD34 cells). Thus, the invention methods for isolating a subpopulation of cells can use a second ligand that is a peptide-specific ligand and a target-specific ligand (Figure 6B). An example of such a peptide-specific ligand is the 9069 antibody disclosed herein, which is specific for CD34 cells and is displaced by the PR34^{™} peptide. Therefore, an antibody such as the 9069 antibody can be used to isolate a CD34 cell and simultaneously isolate a second cell using a ligand specific for the second cell, where the ligand is conjugated to the PR34^{™} peptide.

For example, a population of cells containing CD34 cells, such as bone marrow, can be used to isolate a subpopulation of cells containing CD34 cells and some other cell in the bone marrow population, for example, a mesenchymal stem cell (see Figure 7). Such a subpopulation of bone marrow cells can be particularly useful for transplantation applications. A blood cell population can also be used to isolate a subpopulation containing a T cell, B cell, or NK cell population along with, for example, a CD34 cell or another blood cell type. For example, a T cell, B cell or NK cell exhibiting anti-leukemic activity, or any number of specific cell types, can be isolated along with CD34 cells. Such T cell or B cell subpopulations can be particularly useful in applications for stimulating an immune response, for example, an anti-leukemic immune response, whereas the CD34 cell is responsible for reconstituting the hematopoietic system.

The methods of the invention can be used to isolate any of a number of specific cell types from a population so long as a target-specific ligand is available for the respective cell types. Such a subpopulation of cell types can be, for example, 2 or more, 3 or more, 4 or more, 5 or more, 6 or more, 7 or more, 8 or more, 9 or more, 10 or more, 12 or more, 15 or more, 20 or more cell types from a population.

When isolating a subpopulation of more than one cell type, any number of target-specific ligands can be conjugated to the same peptide capable of releasing a peptide-specific ligand (Figure 6A, when peptide conjugated to ligands 6 and 8 is the same). By conjugating two or more ligands specific for different cell types to the same peptide or by using a peptide-specific ligand that is also a target-specific ligand, the various cell types in the isolated subpopulation can be conveniently released with the same peptide. Alternatively, multiple target-specific ligands can be conjugated to two or more different peptides capable of displacing a peptide-specific ligand and used to isolate multiple cell types that can be released with the different peptides (Figure 6A, when two different peptides are conjugated to ligands 6 and 8).

In addition to using multiple target-specific ligands to isolate multiple cell types from a population, two or more target-specific ligands to the same cell can be used. The use of more than one target-specific ligand to isolate a single type of cell can be useful, for example, for increasing the efficiency of isolating a particular cell type.

Furthermore, if desired, a population or subpopulation of cells can be contacted sequentially using multiple target-specific ligands. For example, a subpopulation of cells can be isolated with a ligand that recognizes several different cells in a population. An initial isolation step using such a target-specific ligand can be used to enrich for a subpopulation containing a particular cell. Such a subpopulation can be released and sequentially contacted with a different target-specific ligand having specificity for a particular cell in the subpopulation. Such a sequential isolation step can be particularly useful when a target-specific ligand that is specific for a single cell type is unavailable. The sequential method can be used to isolate a subpopulation so that a particular cell type is segregated from other cells that also bind the same ligand followed by binding to a target-specific ligand, allowing the use of a target-specific ligand having specificity for several types of cells to isolate a single cell type.

The method of isolating a subpopulation of cells can further include the step of contacting the first cell-conjugate complex and the second cell-second ligand complex with a free form of a displacing peptide, thereby releasing the first cell from the first cell-conjugate complex and the second cell from the second cell-second ligand conjugate. Thus, the methods of the invention can be used to select and release a subpopulation of cells from a population.

The methods of the invention can be used to isolate a subpopulation of cells. In addition to isolating cells simultaneously or with sequential incubation, cells can also be individually isolated by the methods disclosed herein and pooled to obtain a subpopulation of cells.

The invention also provides a method of using a second ligand to negatively select for a cell that can co-purify with a desired cell (Figure 6C). The method includes the steps of contacting a cell population with the peptide-ligand conjugate of the invention wherein the peptide-specific ligand binds to a second cell in the population, thereby forming a cell-conjugate complex; contacting the peptide-ligand conjugate with the peptide-specific ligand; contacting the cell population with a second ligand specific for the second cell, wherein the second ligand is not displaced by the peptide and binds to a distinct site from the peptide-specific ligand; and isolating the cell-conjugate complex. The method can further comprise the step of contacting the cell-conjugate complex with a free form of the peptide, thereby releasing the first cell from the cell-conjugate complex.

The methods of using a second ligand to negatively select for a cell that can co-purify with a desired cell can be useful to enhance the specificity of the cell selection process (see Figure 6C). For example, the CD34-specific antibody 9069 is a peptide-specific ligand that can also function as a target-specific ligand for CD34 on the CD34+ cell type, as described above. In the case where the cell population contains CD34 cells, for example, bone marrow, the CD34 cells (for example, cell type 4 in Figure 6C) will be co-isolated with target cells bound to a target-specific ligand conjugated to the PR34^{™} peptide (for example, cell type 2 in Figure 6C). If it is desired to exclude the CD34 cell type from an isolated subpopulation of cells, another antibody that also binds to CD34 cells but is a non-displaceable antibody, that is, which is not displaced by the PR34^{™} peptide or any other peptide to be used in the reaction, can be included (for example, ligand 16 in Figure 6C). If the non-displaceable ligand is bound to a solid support, cell type 2 can be released whereas cell type 4 remains captured via binding to the non-displaceable target-specific ligand for cell type 4, that is, by ligand 16 (Figure 6C).

The cells can be simultaneously contacted with the non-displaceable ligand or sequentially contacted after displacing with the peptide. The non-displaceable antibody is non-competitive with respect to binding to the displacing peptide. If the non-displaceable ligand is contacted prior to incubation with a displacing peptide, the non-displaceable ligand is non-competitive with respect to the peptide-specific ligand that is also a target-specific ligand. For example, in the case of the 9069 antibody, the non-displaceable ligand binds non-competitively to a CD34 target cell. The non-displaceable ligand can bind to the same molecule as the peptide-specific ligand that is also a target-specific ligand or to a different molecule on the same target cell, so long as the cell to be negatively selected is sufficiently removed from the cell subpopulation.

In general, the second ligand used to negatively select a cell is used in an amount that is sufficient to bind the cells to be negatively selected, depending on the relative affinities of the ligands. The relative amounts can be compensated accordingly based on the relative affinities. For example, if the affinities differ by a factor of 10, the relative amounts can be adjusted to differ by about 10, that is the lower affinity ligand is at 10 times the concentration as the higher affinity ligand, so that approximately an equivalent amount of binding activity is present. In general, the second ligand used to negatively select a cell is used in at least an equivalent amount of binding activity, and will particularly be used in excess so that negatively selected cells are efficiently retained. The second ligand used to negatively select a cell will generally be present in at least about 2-fold, about 3-fold, about 4-fold, about 5-fold, about 6-fold, about 7-fold, about 8-fold, about 9-fold or about 10-fold excess binding activity, and can be present in at least about 50-fold, about 100-fold, about 1000-fold, about 10,000-fold or even about 20,000-fold excess binding activity or greater, if desired.

When isolating a subpopulation of cells using at least two target-specific ligands, the cells are generally isolated on a solid support, for example, beads such as magnetic beads or particles, as disclosed herein. If desired, the target specific-ligands can be bound to the same bead. Alternatively, distinct target-specific ligands can be bound to separate beads, which can be incubated sequentially or simultaneously.

The method of the invention can be used to obtain a substantially pure cell subpopulation comprising CD34 cells and at least one other specifically isolated cell type. As described above, a CD34 cell, which expresses the cD34 antigen, can be isolated by the methods of the invention or other methods and, if desired, co-isolated with a second cell type. As used herein, "substantially pure," when used in reference to a cell or cell subpopulation, refers to an enrichment of a specifically isolated cell relative to an original cell population. The second cell type can be a mesenchymal cell, T cell, B cell, natural killer cell, or facilitating cell, or any type of cell for which a target specific ligand is available for isolating the cell type by the methods disclosed herein. A "facilitating cell" is a tolerance inducing cell (see, for example, Schuchert et al. Nature Med. 6:904-909 (2000)) that induces tolerance towards HLA antigen and can be useful, for example, in graft-versus-host disease or transplant rejection. It is understood that, in addition to subpopulations containing CD34 cells, any combination of cells can be isolated in a cell subpopulation as a useful combination of cells for treating a variety of conditions so long as a target-specific ligand is available for the cell targets to be isolated in the subpopulation (see below).

The substantially pure subpopulation can be enriched for particular cell types by specifically isolating the cells using the methods disclosed herein. A substantially pure subpopulation of cells will generally comprise at least about 10%, at least about 20%, at least about 30%, at least about 40%, at least about 50%, at least about 60%, at least about 70%, at least about 80%, at least about 90%, at least about 95%, at least about 98%, or at least about 99% of the substantially pure subpopulation, and can be essentially 100% of the substantially pure population. It is understood that the specifically isolated cell types in the subpopulation need not be in equal amounts but can be in any ratio so long as each of the specifically isolated cell types is enriched relative to the initial cell population. For example, the methods of the invention can be used to isolate CD34 cells and mesenchymal cells, as described above, and a substantially pure subpopulation of CD34 cells and mesenchymal cells can be from 10% up..to essentially 100% of the substantially pure subpopulation.

The combination of cells in a substantially pure subpopulation can be useful for a variety of therapeutic applications. For example, the combination of CD34 cells and mesenchymal cells can be useful for augmenting engraftment of a patient in need thereof with stem cells or providing the patient with cells of the hematopoietic system. Mesenchymal stem cells can provide stromal, collagen or bone cells and the like, and the CD34 cells can provide hematopoeitic cell function. The combination of CD34 cells and natural killer (NK) cells can be useful, for example, for providing a patient in need thereof with enhanced anti-tumor activity or anti-viral activity. The combination of CD34 cells and T cells or a T cell subset can be useful, for example, for providing enhanced anti-tumor activity, anti-viral activity, immune function, or enhanced response in a transplantation setting.

A response to a condition in an individual can be enhanced using substantially pure cell subpopulations isolated by methods of the invention. Using a substantially pure subpopulation of cells is particularly useful for treating an immuno-compromised individual. Such an individual includes an individual having a congenital defect of the blood forming system or mesenchymal cell system, an individual having cancer, either a cancer that suppresses the immune system such as leukemia or immunosuppression resulting from cancer treatment, or an individual having an autoimmune disease that results in immunosuppression or requires immunosuppresive therapy. As used herein, an individual having immunosuppression has a reduced immune response that can result from a physical stimulus (e.g., X-irradiation), chemical stimulus (e.g., immunosuppressive drugs), or biological stimulus (e.g., anti-lymphocyte serum or congenital defects of the immune system).

A substantially pure subpopulation of cells can be used, for example, to treat an individual who has undergone or will undergo transplantation, which can be organ transplantation or stem cell transplantation. The substantially pure subpopulations of cells can also be used to treat an individual having immunuosuppression. It is thus possible to treat an individual or enhance the individual's response to therapy for treating a condition. As used herein, "enhancing a response to a therapy" refers to any measurable improvement in a sign or symptom associated with a condition. One skilled in the art can readily determine whether an individual exhibits measurable improvement in a sign or symptom associated with a condition, which can be immediate signs or symptoms that are directly measurable in the individual or is reflected in longer life span or a better quality of life.

The substantially pure subpopulations of cells isolated by methods of the invention or any other methods can also be used to provide cells having desired characteristics, for example, the isolation of a functional cell and a feeder cell. A functional cell provides a particular cellular function while a feeder cell provides a stimulus for the functional cell. One such combination of functional and feeder cells that can be isolated by methods of the invention is a CD4 cell, which produces IL-2, and a natural killer (NK) cell, which is activated by IL-2. Thus, the invention provides a combination of CD4 and NK cells, which can function as a feeder cell and a functional cell, respectively, and be used to stimulate an NK cell immune response.

Another exemplary feeder cell and functional cell combination is CD34 cells combined with mesenchymal cells. Mesenchymal cells produce cytokines that stimulate growth of CD34 cells. Thus, in a substantially pure cell subpopulation containing mesenchymal and CD34 cells, mesenchymal cells can function as a feeder cell for CD34 cells, which can function in providing cells of the hematopoietic system. Similarly, stromal cells can also function as a feeder cell for stimulating growth of CD34 cells. It is understood that the methods of the invention can be used to isolate any useful combination of feeder and functional cells using the methods of selecting cells disclosed herein.

A substantially pure subpopulation of cells can also be useful in cell therapy applications. For example, retroviral vectors are commonly used to transduce genes for in vivo cell therapy methods. However, retroviral vectors require that the cells be replicating in order to efficiently infect the cells. Therapeutic methods requiring the use of retroviral vectors therefore use proliferating cells. One method to obtain proliferating cells is to use a substantially pure subpopulation of cells isolated by methods of the invention containing a functional cell and feeder cell combination, where the feeder cell can stimulate growth of the functional cell. For example, a combination of mesenchymal cells and CD34 cells can be used in such a therapeutic application, with the mesenchymal cells providing cytokines that function as a growth stimulus to proliferate CD34 cells. The proliferating CD34 cells in such a subpopulation can thus be transduced with a therapeutic gene and the transduced cells used in in vivo therapeutic methods. Thus, methods of increasing the transduction efficiency of cells for *in vivo* therapeutic applications are disclosed.

There are provided diagnostic systems, preferably in form of a kit for isolating a cell, comprising either a peptide capable of displacing an antibody specific for said peptide from a preformed complex between a peptide-ligand conjugate and the antibody, a cross-linking agent that allows the peptide to be conjugated to the ligand, and said antibody, wherein the antibody is specific for a target molecule on a cell, in a suitable packaging material. For example, the kit can contain the peptide QQGWFP (SEQ ID NO:1); QQGWFPKD (SEQ ID NO:2); QQGWFPKDC (SEQ ID NO.:3), or a combination thereof, and the 9C5 antibody. Invention diagnostic systems are useful for assaying for the presence or absence of a cell type in a sample.

A suitable kit of the invention includes at least one invention peptide and antibody as a separately packaged chemical reagent(s) in an amount sufficient for at least one assay and a cross-linking agent, as disclosed herein, that allows the peptide to be conjugated to any desired ligand. A kit of the invention can also contain a peptide-ligand conjugate of the invention in which the ligand in the conjugate is specific for a cell surface target molecule and which allows the isolation of a particular cell expressing that target molecule. An invention kit can further contain a solid support such as beads to isolate cells, for example, the kit can include magnetic beads.

The contents of the kit of the invention are contained in packaging material, preferably to provide a sterile, contaminant-free environment. In addition, the packaging material contains instructions indicating how the materials within the kit can be employed to displace a prebound peptide-ligand complex with the enclosed peptide or to isolate a particular cell, if appropriate. The instructions for use typically include a tangible expression describing the reagent concentration or at least one assay method parameter, such as the relative amounts of reagent and sample to be admixed, maintenance time periods for reagent/sample admixtures, temperature, buffer conditions, and the like.

The invention additionally provides a method of selecting a cell. The method includes the steps of contacting a cell population with the peptide-specific ligand, wherein the cell population comprises a cell expressing a cell surface fusion polypeptide fused to a peptide, wherein the free form of the peptide is capable of displacing the peptide-specific ligand bound to the fusion polypeptide from a preformed complex between a peptide-ligand conjugate and a second ligand specific for the peptide, thereby forming a cell-peptide ligand complex; and isolating the cell-peptide ligand complex. The method can further include the step of contacting the cell-peptide ligand complex with a free form of the peptide, thereby releasing the cell from the cell-peptide ligand complex.

In addition to using a peptide-ligand conjugate to bind to a target cell, the invention also provides methods of isolating cells that advantageously use peptides capable of displacing a ligand from a preformed complex between a peptide-ligand conjugate and a second ligand specific for the peptide, where the ligand forms a complex with the peptide expressed as a fusion with a molecule expressed on the surface of a cell. In this case, the peptide is expressed as a fusion polypeptide with a cell surface polypeptide, thus bypassing the binding of a peptide-ligand conjugate where the ligand is specific for a target cell. As used herein, a "fusion polypeptide" is one in which at least two heterologous molecules that are not naturally expressed together are expressed as a single translation product. In the case of a peptide fused to a cell surface polypeptide, at least one copy of the peptide is expressed as a fusion with the cell surface polypeptide. If desired, multiple copies of the peptide can be expressed as part of the fusion polypeptide. The peptide is fused to the cell surface polypeptide such that the peptide is expressed on the surface of the cell and accessible to a peptide-specific ligand administered to the cell.

The peptide can be expressed as a fusion with a cell surface polypeptide using well known methods . (Ausubel et al., *supra*, 1999). Methods of isolating cells expressing recombinant peptide-cell surface polypeptide fusions are useful for isolating or enriching for genetically engineered cells and can be used to isolate or enrich for cells useful in therapeutic applications such as gene therapy.

Cells can be isolated by capturing the cells with a solid support such as beads, as described above. Alternatively, cells can be isolated by FACS. The use of FACS to isolate cells is particularly useful when isolating a cell expressing a peptide-surface polypeptide fusion.

It is understood that modifications which do not substantially affect the activity of the various embodiments of this invention are also provided. Accordingly, the following examples are intended to illustrate but not limit the present invention.

### EXAMPLE I

### Synthesis of PR34+^{™} Peptide Conjugate with CD8 Antibody and Binding to SUP-T1 Cells

This example describes the synthesis of PR34+^{™} peptide, conjugation to a CD8 antibody, and binding of the peptide-antibody conjugate to T cells.

The peptide Ac-QQGWFPKD (PR34+^{™}; SEQ ID NO:2) was identified as a peptide capable of displacing a ligand specific for the peptide essentially as described previously (U.S. Patent No. 5,968,753).

The PR34+^{™} peptide was synthesized with a C-terminal Cys residue and covalently coupled to MT-415, an antibody directed against human CD8 to generate the CD8-PR34+ conjugate. The peptide was conjugated to the CD8 antibody using the heterobifunctional linker N-(e-maleimidocaproyloxy) succinimide ester (EMCS). The standard protocol recommended by the manufacturer was used (Pierce Chemical Co.; Rockford IL).

The 9C5 antibody, which is an anti-human CD34 monoclonal antibody, was biotinylated using Biotin Hydrazide (Pierce). The biotinylation reaction was performed following the standard protocol recommended by the manufacturer (Pierce) except that the initial meta-periodate oxidation was performed at room temperature rather than refrigerated.

For flow cytometry experiments, SUP-T1 cells were used. SUP-T1 is a T-lymphoma-derived tumor cell line known to express typical T cell surface markers. The SUP-T1 cells were prepared for flow cytometry and sequentially sensitized with the CD8 antibody-PR34+ peptide (CD8-PR34+) conjugate, biotinylated 9C5, and phycoerythrin-labeled (PE-labeled) streptavidin. The SUP-T1 cells were incubated with either streptavidin-PE; sequentially with the CD8-PR34+ conjugate, biotinylated 9C5, followed by streptavidin-PE; or sequentially with CD8-PR34+ conjugate, biotinylated 9C5, streptavidin-PE, followed by a 15 min incubation with free PR34+^{™} peptide. The SUP-T1 cells were then analyzed by flow cytometry. All incubations were performed in a mixture of Dulbecco's phosphate buffered saline (GibcoBRL), 0.05% azide, 1.0% human serum albumin (PAH). All incubations were for 15 min at room temperature and each was followed by a wash with 1.0 ml PAH. The stained cells were resuspended in 0.25 ml PAH for flow cytometry. SUP-T1 cells (1x10⁶) were stained with 1 µg of either the CD8-9069N or the CD34-biotin. The peptide was used at 5.0 mg/ml.

Figure 2 shows the results of the flow cytometry analysis. Incubation of the SUP-T1 cells with streptavidin-PE is shown and indicated by "isotype." The observed shift in the mean channel fluorescence relative to isotype indicated that the antibody-peptide conjugate was binding with good affinity to the target cells (indicated by "Antibodies + SA-PE"). The addition of free PR34+^{™} peptide (QQGWFPKD; SEQ ID NO:2) eliminated the fluorescent signal, as indicated by the left shift of the peak ("Antibodies + Peptide + SA-PE").

These results demonstrate that the CD8-PR34+ conjugate retains T cell binding activity. These results also demonstrate that the CD8-PR34+ conjugate can bind to biotinylated 9C5 and that the CD8-PR34+ conjugate and biotinylated 9C5 complex retains antigenic affinity, allowing T cells to be bound. These results further demonstrate that the free PR34+^{™} peptide can release the biotinylated 9C5 from the CD8-PR34+ conjugate, thereby allowing release of the T cells.

### EXAMPLE II

### Positive Cell Selection of SUP-T1 Cells Using a Peptide-Antibody Conjugate and Streptavidin Beads

This example shows the positive selection of SUP-T1 cells using the CD8-PR34+ conjugate, biotinylated 9C5 and streptavidin beads.

For positive selection, SUP-T1 cells were incubated under various conditions and rosetted using streptavidin beads. For sensitization, 1x10⁶ SUP-T1 cells in Isolex^{™} Buffer (phosphate buffered saline, 15 mM citrate, 1% human albumin) were sensitized with 1 µg CD8-9069N, washed with 1 ml Isolex^{™} buffer, sensitized with 1 µg CD34-biotin, and washed with 1 ml Isolex^{™} buffer. Both incubations were in a volume of 500 µl for 15 min at room temperature. For rosetting, 15 µl of Streptavidin (SA) coated beads (Dynal) were added to each tube. The tubes were incubated for 30 min at room temperature with mild agitation. For elution, the rosetted cells were washed with Isolex^{™} buffer using a magnet to trap the beads. The beads were then incubated for 15 min with 5 mg/ml PR34+^{™} peptide in a 500 µl volume. The mixture was exposed to the magnet for 2 min, and the supernatant was pipetted off. The beads were washed with Isolex^{™} buffer, and this wash was combined with the earlier supernatant. The total released cells were counted.

In the control reaction (tube 1), SUP-T1 cells were incubated directly with streptavidin beads. For positive selection, SUP-T1 were cells were incubated under two conditions. In the first condition (tube 2), SUP-T1 cells were incubated sequentially with the CD8-PR34+ conjugate, followed by biotinylated 9C5 and then streptavidin beads. In the second condition (tube 3), SUP-T1 cells were incubated with the CD8-PR34+ conjugate, and the streptavidin beads were incubated separately with biotinylated 9C5. Both were washed and then incubated together for 30 min.

Following the rosetting reaction with streptavidin beads, the remaining free cells were removed, and all three reactions were incubated with PR34+^{™} peptide as a release agent for 30 min. The free cells were then collected and quantitated.

As shown in Table 1, SUP-T1 cells incubated with streptavidin beads alone (tube 1) captured only 1% of the SUP-T1 cells. In contrast, about 80% of SUP-T1 cells incubated sequentially with the CD8-PR34+ conjugate followed by biotinylated 9C5 and streptavidin beads (tube 2) were captured. About 44% of SUP-T1 cells were captured when incubated with the CD8-PR34+ conjugate followed by biotinylated 9C5 pre-bound to streptavidin beads (tube 3).

**Table 1. Positive Selection of SUP-T1 Cells.**

| Tube | % SUP-T1 Capture | % SUP-T1 Yield |
|---|---|---|
| 1 | 1 | 6.16 |
| 2 | 80 | 43 |
| 3 | 44 | 31 |

Incubation with free P34+ peptide released the bound SUP-T1 cells captured by the streptavidin beads. The yield of SUP-T1 cells was about 43% for cells captured with sequential incubation with CD8-PR34+ conjugate followed by biotinylated 9C5 and streptavidin beads. The yield of released cells was about 31% for cells treated with CD8-PR34+ conjugate followed by 9C5 pre-bound to streptavidin beads.

These results demonstrate that the CD8-PR34+ conjugate can be used to capture a high percentage of SUP-T1 cells, which express a CD8 antibody receptor. These results further demonstrate that the free PR34+^{™} peptide can be used to displace SUP-T1 cells captured by the CD8-PR34+ conjugate.

### EXAMPLE III

### Positive Selection of CD8+ Cells from a Mononuclear Cell Preparation

This example describes the positive selection of cells expressing CD8 from a population of mononuclear cells.

To test the ability of the CD8-PR34+ conjugate to isolate CD8+ cells from a population, mononuclear cells (MNCs), which contain CD8+ cells, were prepared. Mononuclear cells were collected from human donors. Mononuclear cells were collected from a G-CSF mobilized human donor using a CS-3000^{™} (Baxter) following the protocol of the manufacturer. The starting population of MNCs was 19.42% CD3+/8+. For each rosetting reaction, a quantity of MNCs containing 2x10⁶ CD8+ cells was used. Biotinylated CD8 antibody (MT-415) was biotinylated essentially as described in Example I.

The MNCs were incubated under three conditions. In condition 1, MNCs were incubated with streptavidin beads alone. In condition 2, MNCs were incubated with biotinylated CD8 antibody, followed by streptavidin beads. In condition 3, MNCs were incubated with CD8-PR34+ conjugate, followed by biotinylated 9C5 and streptavidin beads. After incubation with streptavidin beads, the free cells were removed, and the cells attached to streptavidin beads were incubated with free PR34+^{™} peptide. The released cells were counted, and the number of CD8+ cells was evaluated using flow cytometry. The CD8+ cells were quantitated using a combination of CD3 and CD4. CD3 is a pan T cell marker, and CD3+ populations can be further subdivided into mutually exclusive CD4+ and CD8 positive populations. Therefore, CD8+ cells can also be defined as the majority of cells that are CD3+/4-. The incubation conditions were essentially the same as those described in Example II.

As shown in Table 2, about 19% of the CD8+ cells were captured with streptavidin beads alone (condition 1). In contrast, greater than 90% of the CD8+ cells were captured with the biotinylated CD8 antibody (condition 2), demonstrating the efficient binding of CD8+ cells with the anti-CD8 antibody. Similarly, greater than 83% of CD8+ cells were captured with the CD8-PR34+ conjugate (condition 3), demonstrating that the conjugated CD8 antibody retained antigen binding activity that allowed efficient binding of CD8 cells in the mononuclear cell preparation.

**Table 2. Selection of CD8 Cells from a Mononuclear Cell Preparation.**

| Condition | % Capture | % Yield | % Purity |
|---|---|---|---|
| 1 | 19.3 | NA | NA |
| 2 | 90.6 | 2.45 | 12.1 |
| 3 | 83.6 | 16.0 | 61.1 |

Incubation of the CD8+ cells captured with the CD8-PR34+ conjugate with free PR34+^{™} release peptide resulted in a 16% yield of CD8+ cells with 61% purity. The concentration of CD3+/4- cells (i.e., CD8+ cells) was 3.5 fold higher in the released cells than in the starting cell population. However, there was less than 3% yield of CD8+ cells displaced with the free PR34+^{™} release peptide. The low yield of CD8+ cells in condition 2 indicates that mechanical release did not significantly contribute to the release of cells.

These results demonstrate that the CD8-PR34+ conjugate can be used to isolate cells bearing the corresponding receptor, specifically CD8+ cells, from a population of mononuclear cells and can be used to isolate a high percentage of the CD8+ cells present in the mononuclear cell population. These results also demonstrate that the captured cells can be released with free PR34+^{™} peptide, resulting in high purity of CD8+ cells.

### EXAMPLE IV

### Positive Selection of CD2+ T Cells

This example describes the positive selection of CD2+ T cells using a peptide-ligand conjugate.

To test the ability of a PR34+ peptide-conjugate to isolate a different cell type, the PR34+^{™} peptide was coupled to a CD2 antibody (B-E2; IgG2b isotype; Diaclone; Besançon, France) essentially as described in Example I. The PR34+^{™} peptide, also designated 9069N, conjugated to the CD2 antibody was designated CD2-9069N. The biotinylated 9C5 antibody, also known as the 9069 antibody, was designated b-9069.

Either mobilized or non-mobilized MNC preparations (see Example III) were used, with similar results. Cells were incubated with 1 µg or 5 µg of CD2-9069N conjugate for 15 min at room temperature (see Table 3). The cells were then incubated with 1 µg or 10 µg of biotinylated 9C5 antibody (b-9069) and streptavidin beads. Bound cells were subsequently incubated with either buffer or 2.5 mg of free PR34+^{™} peptide (9069N). Incubation conditions were essentially as described in Example II. The released cells were then quantitated and the purity determined. Purity and cell counts were assessed using an anti-CD3 antibody and the True Count flow cytometry quantitation method (Becton Dickenson; San Jose CA).

As shown in Table 3, incubation with the CD2-9069N conjugate captured a high percentage (at least 75%) of CD2+ cells. There appeared to be slightly higher efficiency of capture when incubated with 5 µg of conjugate than 1 µg. These results show that the CD2-9069N conjugate can be used to isolate a high percentage of CD2+ T cells. Incubation with free peptide resulted in release of a high percentage of the captured CD2+ cells, ranging from 50 to 87%. In contrast, incubation with buffer resulted in only a small percentage of the captured cells being released. These results show that the free peptide can specifically release cells captured with a CD2-9069N peptide conjugate. The purity of the cells released by the free peptide was high, ranging from 70 to 89% purity.

**Table 3. Positive Selection of CD2⁺ T cells using CD2-9069N.**

| Experimental Conditions | % T-cells captured | Release Reagents | % T-cells released | % Purity (CD3+) |
|---|---|---|---|---|
| | (15 min./RT) | (30 min./RT) | | |
| CD2-9069N (1 µg) | 75 | 9069N (2.5 mg) | 71 | 89 |
| b-9069 (1 µg) | | | | |
| | | buffer | 15 | 81 |
| CD2-9069N (5 µg) | 87, 93 | 9069N (2.5 mg) | 61, 56 | 88, 81 |
| b-9069 (1 µg) | | | | |
| | | buffer | 20, 8.2 | 76, 66 |
| CD2-9069N (1 µg) | 77,89 | 9069N (2.5 mg) | 87, 65 | 87, 74 |
| b-9069 (10 µg) | | | | |
| | | buffer | 9.0, 5.0 | 69, 61 |
| CD2-9069N (5 µg) | 95 | 9069N (2.5 mg) | 50 | 70 |
| b-9069 (10 µg) | | | | |
| | | buffer | 5.5 | 61 |

These results demonstrate that the CD2-PR34+ peptide conjugate can be used to isolate CD2+ cells, and the free PR34+^{™} peptide can be used to release a high percentage of cells having a high degree of purity. These results also show that the a peptide capable of displacing a ligand specific for the peptide, the peptide/ligand pair PR34+/9C5, can be used to isolate a variety of cells types when the peptide is coupled to a cell-specific ligand.

### EXAMPLE V

### Positive Selection of CD2+ T Cells by Direct Binding to Beads

This example describes positive selection of CD2 T cells by direct binding of a peptide-specific antibody to beads.

The CD2 antibody B-E2 was conjugated to PR34^{™} peptide essentially as described in Example IV. MNCs were incubated essentially as described in Example IV using CD2-PR34 conjugate. The cells were then incubated with the CD34 antibody 9069 coated on M-450 Dynabeads (Dynal). The CD34 antibody coated beads were prapared by absorbing various amounts of anti-CD34 antibody on the bead surface. The beads were prebound with 0, 1, 5, 20, or 50 µg of antibody per 10⁸ beads. As a control, an aliquot of cells was incubated with streptavidin coated Dynabeads.

As shown in Table 4, CD2 cells were efficiently captured by the CD34 antibody directly coated on Dynabeads. The percentage shown is the total of CD3 positive cells, which is a pan T cell marker, as is CD2, used to quantitate the CD2 cells. The percentage of cells captured was similar for each amount of antibody tested. The percentage of cells captured was about 60 to 70%, only somewhat lower than the 80% capture observed with streptavidin beads. In the streptavidin coated Dynabead control, biotinylated 9069 antibody (9C5) was used.

**Table 4. T Cell Selection Using CD2-PR34 Conjugate and Capture by Direct Binding of Antibody to Dynabeads.**

| Experimental Condition* | Release Agent*** | %Capture | %Yield | %Purity |
|---|---|---|---|---|
| 50 µg | PR34+^{™} | 64.5 | 57.5 | 91.4 |
| | (2.5 mg) | | | |
| | buffer | 64.5 | 26.0 | 89.1 |
| 20 µg | PR34+^{™} | 61.7 | 47.4 | 91.8 |
| | (2.5 mg) | | | |
| | buffer | 61.7 | 0.2 | 82.1 |
| 5 µg | PR34+^{™} | 72.0 | 68.9 | 91.3 |
| | (2.5 mg) | | | |
| | buffer | 72.0 | 23.5 | 88.3 |
| 1 µg | PR34+^{™} | 73.0 | 55.9 | 92.4 |
| | (2.5 mg) | | | |
| | buffer | 73.0 | 7.6 | 71.7 |
| 0 µg | PR34+^{™} | 2.88 | ND (low) | ND |
| | (2.5 mg) | | | |
| SA** | PR34+^{™} | 80.4 | 81.5 | 94.5 |
| | (2.5 mg) | | | |

| | | | | |
|---|---|---|---|---|
| % is of the total CD3 positive cells. *Dynal M-450 Dynabeads were prepared by absorbing varying amounts of anti-CD34 antibody on the bead surface. The number refers to µg antibody per 10⁸ beads. **Streptavin coated Dynabead control. ***PR34+^{™} CD34 release agent used to displace the CD2-PR34 conjugate from the CD34 antibody. | | | | |

Incubation of the beads with free PR34+^{™} peptide resulted in about 50% or greater yield of CD2 cells. The purity of the cells released by free peptide was greater than 90%, which was comparable to the purity of cells captured and released from streptavidin beads (see Table 4).

These results demonstrate that cells bound by a peptide-ligand conjugate can be efficiently captured by directly binding a peptide-specific antibody to beads. The captured cells can also be efficiently released from the beads with a high purity.

### EXAMPLE VI

### Release of Cells Captured with CD2 Antibody Using Various Peptides

This example describes the displacement of cells bound to anti-CD2 antibody by various peptides.

Peptides having affinity for the CD2 monoclonal antibody B-E2 were isolated by phage display biopanning. Various peptides were tested as competitors for binding of T cells to the anti-CD2 antibody B-E2. A competition assay was performed using 1 mM of each peptide. The peptides were at 2x10⁷ fold molar excess relative to the competitor CD2 antigen and at 6x10⁶ fold molar excess relative to anti-CD2 antibody. Competition was measured using a FACS competition assay (see Figure 3).

For the competition assay, 0.5 ml of a 1 mM solution of various peptides was added to 1x10⁶ peripheral blood mononuclear cells (MNC) followed by the addition of 100 µl of 0.02 µg/ml of CD2 antibody (B-E2) and incubated for 15 min at room temperature. Cells were washed once in lysing reagent (155 mM ammonium chloride, 10 mM potassium bicarbonate, and 0.1 mM EDTA) and twice in 2 ml of PAH (DPBS, 1% human serum albumin, 0.02% NaN₃). 1 µl of goat F(ab')2 fragment anti-mouse IgG (heavy + light)-phycoerythrin conjugated (GAM-PE; Immunotech; Miami FL) was added to each sample and incubated for 10 min at room temperature. Cells were washed twice in 2 ml of PAH. The final cell pellet was resuspended in 0.25 ml of 0.5% paraformaldehyde. The samples were analyzed by flow cytometry.

The results of the competition assay are shown in Figure 3. A variety of peptides exhibited competitive binding activity. Cells treated with the peptides indicated by "*" appeared apoptotic.

Several of the peptides were further tested for release activity. Briefly, the FACS release assay was performed by incubating 1x10⁶ MNC with 100 µl of 0.02 pg/ml of CD2 antibody for 15 min at room temperature. Cells were washed once with 2 ml of lysing reagent and twice with 2 ml of PAH solution. 0.5 ml of a 1 mM or 5 mM peptide stock was added to the cell pellet and incubated at room temperature for 30 min. Cells were washed twice in 2 ml of PAH. 1 µl of GAM-PE was added to each sample and incubated for 10 min at room temperature. Cells were washed twice in 2 ml of PAH. The final cell pellet was resuspended in 0.25 ml of 0.5% paraformaldehyde. The samples were analyzed by flow cytometry.

In the release assay, either 1mM or 5 mM peptide (1 mM peptide except where indicated to be 5 mM in Figure 4). In some cases, the release assay was performed in the presence of 1 mM dithiothreitol (DTT). The assay was performed with two different batches of donor cells (second batch of donor cells designated with "*" in Figure 4). The results of the release assay are shown in Figure 4. The cyclic peptide RCRRPATTPACR appeared to have the highest release activity.

These results demonstrate that a variety of peptides can function to displace a CD2 antibody.

### EXAMPLE VII

### Positive Selection of Camma Cells Using Pan-O-5 Monoclonal Antibody

This example describes positive selection Camma cells, a breast cell line, using Pan-0-5 monoclonal antibody, which can select a breast cell line.

To prepare antibody-coated beads, Dynabeads (Dynal) were washed with Dulbecco's phosphate buffered saline (D-PBS). Washed beads were coated with 50 µg anti-CD34 monoclonal antibody per 10⁸ beads for 24 hours at 4°C. The beads were washed twice with Isolex buffer and incubated with 1% albumin in the Isolex buffer for 24 hours at 4°C.

To synthesize the Pan-O-5-PR34 conjugate, the Pan-O-5 monoclonal antibody was incubated with N-(ε-maleimidocaproyloxy)succinimide ester for 30 min at room temperature to generate an antibody-linker complex. The antibody-linker complex was washed twice with D-PBS using an Amicon spin column (30KD; Millipore Corp.; Bedford MA). The Pan-O-5-antibody-linker complex was incubated with PR34^{™} peptide for 30 min at room temperature. The Pan-0-5-linker-PR34 antibody complex was washed twice with D-PBS using an Amicon spin column. The concentration of the conjugate was determined by absorbance at 280 nm.

Cells (18 x 10⁶) were incubated with Pan-O-5-linker-PR34 antibody (5 µg/ml) for 15 min. The cell-antibody complex was washed twice with Isolex buffer and incubated with anti-CD34-coated Dynabeads (5 beads per cell) for 30 min at room temperature. The Dynabeads were isolated with a magnet, and unbound cells were removed. The bead-cell fraction was incubated with PR34^{™} peptide (5 mg/ml) for 30 min at room temperature. Released cells were separated from unreleased cells using a magnet. The results are shown in Figure 5 (N=1).

As shown in Figure 5, the Pan-O-5-linker-PR34 antibody efficiently captured greater than 90% of the Camma cells. Greater than 75% of cells were released using PR34^{™} peptide. The yield of Camma cells was greater than 70%.

These results demonstrate that the peptide-ligand conjugate method for positively selecting cells can be used with a variety of antibodies specific for various target cells, including the Pan-O-5 antibody, which can select breast cancer cells.

## Claims

1. A peptide-ligand conjugate comprising a peptide conjugated to a first ligand specific for a particular cell type, wherein a free form of said peptide is capable of displacing a second ligand specific for said peptide from a preformed complex between a peptide-ligand conjugate and a second ligand specific for the peptide, wherein the peptide-specific ligand is specific for a target molecule on a cell, with the proviso that the "peptide-ligand conjugate" is not a fusion polypeptide in which the peptide is linearly linked to the ligand via the peptide bond to form a single, linear polypeptide.

2. The peptide-ligand conjugate of claim 1, wherein said first ligand specifically binds a target molecule on a cell surface.

3. The peptide-ligand conjugate of claim 2, wherein said target molecule is a cell surface receptor.

4. The peptide-ligand conjugate of claim 1, wherein said first ligand is an antibody.

5. The peptide-ligand conjugate of claim 4, wherein said antibody is an anti-CD8 antibody or an anti-CD2 antibody.

6. The peptide-ligand conjugate of claim 1, wherein said second ligand is an antibody.

7. The peptide-ligand conjugate of claim 1, wherein said second ligand specifically binds CD34.

8. The peptide-ligand conjugate of claim 7, wherein said second ligand is monoclonal antibody 9C5.

9. The peptide conjugate of claim 1, wherein said peptide comprises an amino acid sequence selected from the group consisting of QQGWFP (SEQ ID NO: 1); QQGWFPKD (SEQ ID NO:2); and QQGWFPKDC (SEQ ID NO:3).

10. The peptide-ligand conjugate of claim 9, wherein said peptide is acylated.

11. A method of selecting a cell, comprising the steps of:
(a) contacting a cell population with the peptide-ligand conjugate of any of claims 1 to 10, thereby forming a cell-conjugate complex;
(b) contacting said peptide-ligand conjugate with said second ligand specific for the peptide, wherein the peptide-specific ligand is specific for a target molecule on a cell; and
(c) isolating said cell-conjugate complex.

12. The method of claim 11, further comprising the step of:
(d) contacting said cell-conjugate complex with a free form of said peptide, thereby releasing said cell from said cell-conjugate complex.

13. The method of claim 11, wherein said first ligand specifically binds a target molecule on the surface of said cell.

14. The method of claim 12, wherein said free form of said peptide comprises the amino acid sequence QQGWFP (SEQ ID NO:1) or QQGWFPKD (SEQ ID NO:2)

15. The method of claim 12, wherein said peptide is acylated.

16. The method of claim 11, wherein said second ligand is a CD34-specific antibody.

17. The method of claim 11, wherein said cell is a stem cell.

18. The method of claim 17, wherein said stem cell is a blood-generated stem cell.

19. The method of claim 18, wherein said blood-generated stem cell is a hematopoietic stem cell.

20. The method of claim 17, wherein said stem cell is a mesenchymal stem cell, a neuronal stem cell or a muscle stem cell.

21. The method of claim 11, wherein said cell is a dendritic cell.

22. The method of claim 11, wherein said cell is a T-cell.

23. The method of claim 11, wherein said cell is a hematopoietic cell.

24. The method of claim 22, wherein said cell is a CD34+ cell.

25. The method of claim 11, wherein said cell is a tumor cell.

26. The method of claim 25, wherein said tumor cell is a cell selected from the group consisting of breast, prostate, colon, lung, lymphoma, myeloma, melanoma, leukemia, ovarian, pancreatic, adrenal, renal cell carcinoma, cervical, hepatic, stomach, and neuroblastoma.

27. A method of enriching for a cell from a cell population, comprising the method of any of claims 11-26.

28. The method of claim 27, wherein said cell is a T-cell, a naive T cell, a memory T cell, or a natural killer cell.

29. A kit for isolating a cell, comprising either a peptide capable of displacing an antibody specific for said peptide from a preformed complex between a peptide-ligand conjugate and the antibody, a cross-linking agent that allows the peptide to be conjugated to the ligand, and said antibody, wherein the antibody is specific for a target molecule on a cell, or the peptide-ligand-conjugate of any of claims 1-10 and said antibody, wherein the antibody is specific for a target molecule on a cell.

30. The kit of claim 29, wherein said peptide comprises an amino acid sequence selected from the group consisting of QQGWFP (SEQ ID NO:1); QQGWFPKD (SEQ ID NO:2); and QQGWFPKDC (SEQ ID NO:3).

31. The kit of claim 29, wherein said peptide comprises the amino acid sequence QQGWFPKDC (SEQ ID NO:1).

32. The kit of claim 30, wherein said peptide is acylated.

33. The kit of claim 29, wherein said antibody specifically binds CD34.

34. The kit of claim 33, wherein said antibody is 9C5.

35. The kit of claim 29, comprising said peptide-ligand conjugate and said peptide.

36. A method of selecting a cell, comprising the steps of:
(a) contacting a cell population with a peptide-specific ligand, wherein said cell population comprises a cell expressing a cell surface polypeptide fused to a peptide, wherein the free form of said peptide is capable of displacing said peptide-specific ligand bound to said fusion polypeptide from a preformed complex between the peptide and a ligand specific for the peptide, and wherein the peptide-specific ligand is specific for a target molecule on a cell, thereby forming a cell-peptide ligand complex; and
(b) isolating said cell-peptide ligand complex.

37. The method of claim 36, further comprising the step of:
(c) contacting said cell-peptide ligand complex with a free form of said peptide, thereby releasing said cell from said cell-peptide ligand complex.

## Patentansprüche

1. Ein Peptid-Liganden-Konjugat, umfassend ein mit einem ersten für einen bestimmten Zelltyp spezifischen Liganden konjugiertes Peptid, wobei eine freie Form des Peptids dazu in der Lage ist, einen zweiten für das Peptid spezifischen Liganden aus einem vorher gebildeten Komplex zwischen einem Peptid-Liganden-Konjugat und einem zweiten für das Peptid spezifischen Liganden zu ersetzen, wobei der Peptid-spezifische Ligand spezifisch für ein Zielmolekül auf einer Zelle ist,
unter der Voraussetzung, dass das "Peptid-Liganden-Konjugat" kein Fusionspolypeptid ist, in dem das Peptid über eine Peptidbindung linear mit dem Liganden verbunden ist, um ein einzelnes lineares Polypeptid zu bilden.

2. Das Peptid-Liganden-Konjugat nach Anspruch 1, **dadurch gekennzeichnet, dass** der erste Ligand spezifisch ein Zielmolekül auf einer Zelloberfläche bindet.

3. Das Peptid-Liganden-Konjugat nach Anspruch 2, **dadurch gekennzeichnet, dass** das Zielmolekül ein Zelloberflächenrezeptor ist.

4. Das Peptid-Liganden-Konjugat nach Anspruch 1, **dadurch gekennzeichnet, dass** der erste Ligand ein Antikörper ist.

5. Das Peptid-Liganden-Konjugat nach Anspruch 4, **dadurch gekennzeichnet, dass** der Antikörper ein anti-CD8 Antikörper oder ein anti-CD2 Antikörper ist.

6. Das Peptid-Liganden-Konjugat nach Anspruch 1, **dadurch gekennzeichnet, dass** der zweite Ligand ein Antikörper ist.

7. Das Peptid-Liganden-Konjugat nach Anspruch 1, **dadurch gekennzeichnet, dass** der zweite Ligand spezifisch an CD34 bindet.

8. Das Peptid-Liganden-Konjugat nach Anspruch 7, **dadurch gekennzeichnet, dass** der zweite Ligand der monoklonale Antikörper 9C5 ist.

9. Das Peptid-Liganden-Konjugat nach Anspruch 1, **dadurch gekennzeichnet, dass** das Peptid eine Aminosäuresequenz ausgewählt aus der Gruppe bestehend aus QQGWFP (SEQ ID NO: 1), QQGWFPKD (SEQ ID NO: 2) und QQGWFPKDC (SEQ ID NO: 3) umfasst.

10. Das Peptid-Liganden-Konjugat nach Anspruch 9, **dadurch gekennzeichnet, dass** das Peptid acyliert ist.

11. Ein Verfahren zur Selektion einer Zelle, Schritte umfassend, bei denen man:
(a) eine Zellpopulation mit dem Peptid-Liganden-Konjugat nach einem der Ansprüche 1 bis 10 in Kontakt bringt und dadurch einen Zell-Konjugat-Komplex bildet;
(b) das Peptid-Liganden-Konjugat mit dem zweiten für das Peptid spezifischen Liganden in Kontakt bringt, wobei der Peptid-spezifische Ligand spezifisch für ein Zielmolekül auf einer Zelle ist; und
(c) den Zell-Konjugat-Komplex isoliert.

12. Verfahren nach Anspruch 11, ferner den Schritt umfassend, dass man:
(d) den Zell-Konjugat-Komplex mit einer freien Form des Peptids in Kontakt bringt, wodurch die Zelle aus dem Zell-Konjugat-Komplex freigesetzt wird.

13. Verfahren nach Anspruch 11, **dadurch gekennzeichnet, dass** der erste Ligand spezifisch an ein Zielmolekül auf der Oberfläche der Zelle bindet.

14. Verfahren nach Anspruch 12, **dadurch gekennzeichnet, dass** die freie Form des Peptids die Aminosäuresequenz QQGWFP (SEQ ID NO: 1) oder QQGWFPKD (SEQ ID NO: 2) umfasst.

15. Verfahren nach Anspruch 12, **dadurch gekennzeichnet, dass** das Peptid acyliert ist.

16. Verfahren nach Anspruch 11, **dadurch gekennzeichnet, dass** der zweite Ligand ein für CD34 spezifischer Antikörper ist.

17. Verfahren nach Anspruch 11, **dadurch gekennzeichnet, dass** der die Zelle eine Stammzelle ist.

18. Verfahren nach Anspruch 17, **dadurch gekennzeichnet, dass** die Stammzelle eine aus Blut hergestellte Stammzelle ist.

19. Verfahren nach Anspruch 18, **dadurch gekennzeichnet, dass** die aus Blut hergestellte Stammzelle eine hämatopoietische Stammzelle ist.

20. Verfahren nach Anspruch 17, **dadurch gekennzeichnet, dass** die Stammzelle eine mesenchymale Stammzelle, eine neuronale Stammzelle oder eine Muskel-Stammzelle ist.

21. Verfahren nach Anspruch 11, **dadurch gekennzeichnet, dass** die Zelle eine dendritische Zelle ist.

22. Verfahren nach Anspruch 11, **dadurch gekennzeichnet, dass** die Zelle eine T-Zelle ist.

23. Verfahren nach Anspruch 11, **dadurch gekennzeichnet, dass** die Zelle eine hämatopoietische Zelle ist.

24. Verfahren nach Anspruch 22, **dadurch gekennzeichnet, dass** die Zelle eine CD34+ Zelle ist.

25. Verfahren nach Anspruch 11, **dadurch gekennzeichnet, dass** die Zelle eine Tumorzelle ist.

26. Verfahren nach Anspruch 25, **dadurch gekennzeichnet, dass** die Tumorzelle eine Zelle ausgewählt aus der Gruppe bestehend aus Brust-, Prostata-, Kolon-, Lungen-, Lymphom-, Myelom-, Melanom-, Leukämie-, Ovar-, pankreatischen, adrenalen, Nierenzellkarzinom-, zervikalen, hepatischen, Magen- und Neuroblastomzellen ist.

27. Verfahren zum Anreichern einer Zelle aus einer Zellpopulation, umfassend das Verfahren nach einem der Ansprüche 11 bis 26.

28. Verfahren nach Anspruch 27, **dadurch gekennzeichnet, dass** die Zelle eine T-Zelle, eine naive T-Zelle, eine Gedächtnis-T-Zelle oder eine Natürliche Killer T-Zelle ist.

29. Ein Kit zur Isolierung einer Zelle, umfassend entweder ein Peptid, welches in der Lage ist, einen für das Peptid spezifischen Antikörper aus einem vorher gebildeten Komplex zwischen einem Peptid-Liganden-Konjugat und dem Antikörper zu ersetzen, ein vernetzendes Agens, welches es erlaubt, das Peptid mit dem Liganden zu konjugieren, und den Antikörper, wobei der Antikörper spezifisch für ein Zielmolekül auf einer Zelle ist, oder das Peptid-Liganden-Konjugat nach einem der Ansprüche 1 bis 10 und den Antikörper, wobei der Antikörper spezifisch für ein Zielmolekül auf einer Zelle ist.

30. Kit nach Anspruch 29, **dadurch gekennzeichnet, dass** das Peptid eine Aminosäuresequenz ausgewählt aus der Gruppe bestehend aus QQGWFP (SEQ ID NO: 1), QQGWFPKD (SEQ ID NO: 2) und QQGWFPKDC (SEQ ID NO: 3) umfasst.

31. Kit nach Anspruch 29, **dadurch gekennzeichnet, dass** das Peptid die Aminosäuresequenz QQGWFPKDC (SEQ ID NO: 1) umfasst.

32. Kit nach Anspruch 30, **dadurch gekennzeichnet, dass** das Peptid acyliert ist.

33. Kit nach Anspruch 29, **dadurch gekennzeichnet, dass** der Antikörper spezifisch an CD34 bindet.

34. Kit nach Anspruch 33, **dadurch gekennzeichnet, dass** der Antikörper 9C5 ist.

35. Kit nach Anspruch 29, umfassend das Peptid-Liganden-Konjugat und das Peptid.

36. Verfahren zur Selektion einer Zelle, Schritte umfassend, bei denen man:
(a) eine Zellpopulation mit einem Peptid-spezifischen Liganden in Kontakt bringt, wobei die Zellpopulation eine Zelle umfasst, die ein mit einem Peptid fusioniertes Zelloberflächenpolypeptid exprimiert, wobei die freie Form des Peptids dazu in der Lage ist, den an das Fusionspolypeptid gebundenen Peptid-spezifischen Liganden aus einem vorher geformten Komplex zwischen dem Peptid und einem für das Peptid spezifischen Liganden zu ersetzen, und wobei der Peptid-spezifische Ligand spezifisch für ein Zielmolekül auf einer Zelle ist, wodurch ein Zell-Peptid-Liganden-Komplex gebildet wird; und
(b) den Zell-Peptid-Liganden-Komplex isoliert.

37. Verfahren nach Anspruch 36, ferner den Schritt umfassend, dass man:
(c) den Zell-Peptid-Liganden-Komplex mit einer freien Form des Peptids in Kontakt bringt, wobei die Zelle aus dem Zell-Peptid-Liganden-Komplex freigesetzt wird.

## Revendications

1. Conjugué de peptide-ligand comprenant un peptide conjugué à un premier ligand spécifique pour un type particulier de cellules, dans lequel une forme libre dudit peptide est capable de déplacer un second ligand spécifique pour ledit peptide à partir d'un complexe préformé entre un conjugué de peptide-ligand et un second ligand spécifique pour le peptide, dans lequel le ligand spécifique pour le peptide est spécifique pour une molécule cible sur une cellule, à condition que le « conjugué de peptide-ligand » ne soit pas un polypeptide de condensation dans lequel le peptide est lié de façon linéaire au ligand au moyen de la liaison peptidique pour former un polypeptide linéaire unique.

2. Conjugué de peptide-ligand selon la revendication 1, dans lequel ledit premier ligand lie spécifiquement une molécule cible sur une surface cellulaire.

3. Conjugué de peptide-ligand selon la revendication 2, dans lequel ladite molécule cible est un récepteur de surface cellulaire.

4. Conjugué de peptide-ligand selon la revendication 1, dans lequel ledit premier ligand est un anticorps.

5. Conjugué de peptide-ligand selon la revendication 4, dans lequel ledit anticorps est un anticorps anti-CD8 ou un anticorps anti-CD2.

6. Conjugué de peptide-ligand selon la revendication 1, dans lequel ledit second ligand est un anticorps.

7. Conjugué de peptide-ligand selon la revendication 1, dans lequel ledit second ligand lie spécifiquement CD34.

8. Conjugué de peptide-ligand selon la revendication 7, dans lequel ledit second ligand est un anticorps monoclonal 9C5.

9. Conjugué peptidique selon la revendication 1, dans lequel ledit peptide comprend une séquence d'aminoacides choisie dans l'ensemble constitué de QQGWFP (SEQ ID NO:1) ; QQGWFPKD (SEQ ID NO:2) ; et QQGWFPKDC (SEQ ID NO:3).

10. Conjugué de peptide-ligand selon la revendication 9, dans lequel ledit peptide est acylé.

11. Procédé de sélection d'une cellule, comprenant les étapes consistant à :
(a) amener une population de cellules au contact du conjugué de peptide-ligand selon l'une quelconque des revendications 1 à 10, formant ainsi un complexe de cellules-conjugué ;
(b) amener ledit conjugué de peptide-ligand au contact dudit second ligand spécifique pour le peptide, le ligand spécifique pour le peptide étant spécifique pour une molécule cible sur une cellule ; et
(c) isoler ledit complexe de cellules-conjugué.

12. Procédé selon la revendication 11, comprenant en outre l'étape consistant à :
(d) amener ledit complexe de cellules-conjugué au contact d'une forme libre dudit peptide, libérant ainsi ladite cellule à partir dudit complexe de cellules-conjugué.

13. Procédé selon la revendication 11, dans lequel ledit premier ligand lie spécifiquement une molécule cible sur la surface de ladite cellule.

14. Procédé selon la revendication 12, dans lequel ladite forme libre dudit peptide comprend la séquence d'aminoacides QQGWFP (SEQ ID NO:1) ou QQGWFPKD (SEQ ID NO:2).

15. Procédé selon la revendication 12, dans lequel ledit peptide est acylé.

16. Procédé selon la revendication 11, dans lequel ledit second ligand est un anticorps spécifique de CD34.

17. Procédé selon la revendication 11, dans lequel ladite cellule est une cellule souche.

18. Procédé selon la revendication 17, dans lequel ladite cellule souche est une cellule souche générée dans le sang.

19. Procédé selon la revendication 18, dans lequel ladite cellule souche générée dans le sang est une cellule souche hématopoïétique.

20. Procédé selon la revendication 17, dans lequel ladite cellule souche est une cellule souche mésenchymateuse, une cellule souche neuronale ou une cellule souche musculaire.

21. Procédé selon la revendication 11, dans lequel ladite cellule est une cellule dendritique.

22. Procédé selon la revendication 11, dans lequel ladite cellule est une cellule T.

23. Procédé selon la revendication 11, dans lequel ladite cellule est une cellule hématopoïétique.

24. Procédé selon la revendication 22, dans lequel ladite cellule est une cellule CD34+.

25. Procédé selon la revendication 11, dans lequel ladite cellule est une cellule tumorale.

26. Procédé selon la revendication 25, dans lequel ladite cellule tumorale est une cellule choisie dans l'ensemble constitué du sein, de la prostate, du côlon, du poumon, d'un lymphome, d'un myélome, d'un mélanome, d'une leucémie, de l'ovaire, de cellules pancréatiques, de la glande surrénale, d'un carcinome des cellules rénales, de cellules cervicales, hépatiques, de l'estomac et d'un neuroblastome.

27. Procédé d'enrichissement pour une cellule à partir d'une population de cellules, comprenant le procédé selon l'une quelconque des revendications 11 à 26.

28. Procédé selon la revendication 27, dans lequel ladite cellule est une cellule T, une cellule T naïve, une cellule T à mémoire ou une cellule tueuse naturelle.

29. Kit pour isoler une cellule, comprenant un peptide capable de déplacer un anticorps spécifique pour ledit peptide à partir d'un complexe préformé entre un conjugué de peptide-ligand et l'anticorps, un agent de réticulation qui permet au peptide d'être conjugué au ligand, et ledit anticorps, dans lequel l'anticorps est spécifique pour une molécule cible sur une cellule, ou le conjugué de peptide-ligand selon l'une quelconque des revendications 1 à 10 et ledit anticorps, dans lequel l'anticorps est spécifique pour une molécule cible sur une cellule.

30. Kit selon la revendication 29, dans lequel ledit peptide comprend une séquence d'aminoacides choisis dans l*'*ensemble constitué de QQGWFP (SEQ ID NO:1) ; QQGWFPKD (SEQ ID NO:2) ; et QQGWFPKDC (SEQ ID NO:3).

31. Kit selon la revendication 29, dans lequel ledit peptide comprend la séquence d'aminoacides QQGWFPKDC (SEQ ID NO:1).

32. Kit selon la revendication 30, dans lequel ledit peptide est acylé.

33. Kit selon la revendication 29, dans lequel ledit anticorps lie spécifiquement CD34.

34. Kit selon la revendication 33, dans lequel ledit anticorps est 9C5.

35. Kit selon la revendication 29, comprenant ledit conjugué de peptide-ligand et ledit peptide.

36. Procédé pour sélectionner une cellule, comprenant les étapes consistant à :
(a) amener une population de cellules au contact d'un ligand spécifique pour le peptide, ladite population de cellules comprenant une cellule exprimant un polypeptide de surface cellulaire condensé avec un peptide, la forme libre dudit peptide étant capable de déplacer ledit ligand spécifique pour le peptide et lié audit polypeptide de condensation à partir d'un complexe préformé entre le peptide et un ligand spécifique pour le peptide, et le ligand spécifique pour le peptide étant spécifique pour une molécule cible sur une cellule, formant ainsi un complexe de cellules-peptide-ligand ; et
(b) isoler ledit complexe de cellules-peptide-ligand.

37. Procédé selon la revendication 36, comprenant en outre l'étape consistant à :
(c) amener ledit complexe de cellules-peptide-ligand au contact d'une forme libre dudit peptide, libérant ainsi ladite cellule à partir dudit complexe de cellules-peptide-ligand.
